(19) Europäisches Patentamt
European Patent Office
Office européen des brevets

(11) EP 2 989 985 A1

(12) **EUROPEAN PATENT APPLICATION**

(43) Date of publication:
**02.03.2016 Bulletin 2016/09**

(51) Int Cl.:
***A61B 8/00*** *(2006.01)* ***A61B 8/08*** *(2006.01)*

(21) Application number: **15182524.7**

(22) Date of filing: **26.08.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **26.08.2014 PCT/JP2014/072247**
**26.08.2014 PCT/JP2014/072250**
**26.08.2014 PCT/JP2014/072251**

(71) Applicant: **Otsuka Medical Devices Co., Ltd.**
**Minato-ku**
**Tokyo 108-0075 (JP)**

(72) Inventors:
- **YOSHIMURA, Yasuo**
**Sagamihara City, Kanagawa 252-0237 (JP)**
- **SUMI, Akira**
**Sagamihara City, Kanagawa 252-0237 (JP)**
- **SHIRASAKI, Isao**
**Sagamihara City, Kanagawa 252-0237 (JP)**

(74) Representative: **von Kreisler Selting Werner - Partnerschaft**
**von Patentanwälten und Rechtsanwälten mbB**
**Deichmannhaus am Dom**
**Bahnhofsvorplatz 1**
**50667 Köln (DE)**

# (54) ULTRASONIC URINE VOLUME MEASURING INSTRUMENT, AND URINE VOLUME MANAGEMENT DATA GENERATING METHOD USING ULTRASONIC URINE VOLUME MEASURING INSTRUMENT

(57) [Object] To provide an ultrasonic urine volume measuring instrument that can highly accurately measure urine volume in the bladder and its temporal changes as needed and that reduces power consumption.

[Solution] In a ultrasonic urine volume measuring instrument 1 including a ultrasound probe 10, a ultrasound controller 21 that controls transmission and reception of ultrasound and its reflected waves, and an arithmetic unit 24 that calculates urine volume in the bladder on the basis of the reflected waves every measurement cycle, the arithmetic unit 24 includes a comparator 24d that compares the calculated urine volume in the bladder with a certain first threshold. The ultrasound measurement cycle becomes a longer cycle in the case where the urine volume in the bladder compared by the comparator 24d is less than the first threshold than that in the case where the urine volume is greater than or equal to the first threshold. The ultrasonic urine volume measuring instrument 1 includes a timer unit 28 capable of causing, in the case where the urine volume in the bladder compared by the comparator is less than the first threshold, the arithmetic unit to enter a sleep state during each measurement cycle, and causing the arithmetic unit to recover every measurement cycle.

EP 2 989 985 A1

## Description

[Technical Field]

The present invention relates to an ultrasonic urine volume measuring instrument that measures urine volume in the bladder using ultrasound, and a urine volume management data generating method using the ultrasonic urine volume measuring instrument.

[Background Art]

Hitherto, as a measuring instrument that uses ultrasound to measure urine volume in the bladder, for example, as described in PTL 1 (Japanese Patent No. 4677615), one that uses ultrasound A-mode to chronologically measure urine volume in the bladder on the basis of the amplitude and time of waves reflected from the front and back walls of the bladder is known.

In such a measuring instrument that uses ultrasound to measure urine volume in the bladder, as a method of arranging the measuring instrument on the stomach of a measurement subject, for example, as described in PTL 2 (Japanese Unexamined Patent Application Publication No. 2009-512532), a method is disclosed in which an image of the bladder is generated using ultrasound B-mode that uses many ultrasound devices, and the positions of the ultrasound devices are adjusted to be optimal by looking at the generated image. However, using this method takes a time for adjustment since the position adjustment is done while looking at the bladder image based on the ultrasound B-mode measurement. In addition, this can only be implemented by a large-size ultrasonic urine volume measuring instrument that has many ultrasound devices.

In addition, PTL 3 (Japanese Unexamined Patent Application Publication No. 2000-210286) discloses that many ultrasound devices arranged in a matrix are fixed, and an index value is obtained using a column of ultrasound devices with the highest sensitivity among the many ultrasound devices. However, this ultrasonic urine volume measuring instrument cannot adjust the positions or angles of the ultrasound devices. Since the ultrasound devices are selected only on the basis of the reception sensitivity, not on the basis of the actually measured urine volume, the ultrasound devices cannot be arranged at the most appropriate positions or angles.

As an ultrasonic urine volume measuring instrument that changes its measurement frequency, for example, PTL 4 (US Patent No. 6565512) discloses one that monitors urine volume in the bladder using many ultrasound devices arranged in two dimensions, that produces an alarm in the case where the urine volume exceeds a certain value, and that increases a monitoring frequency in the case where the urine volume exceeds 75% of the certain value.

Furthermore, in PTL 5 (Japanese Unexamined Patent Application Publication No. 2007-508857), urine volume in the bladder is measured using frequency sweep. Here, it is disclosed that, when the bladder is full, a higher sampling rate is used.

However, neither PTL 4 nor 5 describes a specific configuration for changing the measurement frequency.

In addition, as one that generates a urination diary using an ultrasonic urine volume measuring instrument, PTL 6 (Japanese Unexamined Patent Application Publication No. 2011-183142) describes that the volume of the bladder is obtained using twelve ultrasound devices, and a urination diary is automatically recorded by a data processing apparatus. However, PTL 6 does not describe a specific configuration for recording a urination diary.

In addition, PTL 7 (Japanese Unexamined Patent Application Publication No. 2014-23813) discloses that, in the case where a person who is a measurement subject at least feels urgency toward micturition and/or feels that the bladder is not completely empty, that complaint information is input by the person who is a measurement subject and is stored in urination storage means. However, although the complaint information is recorded in PTL 7, that recording can only be checked afterwards, and that does not directly help urination of a person who is a measurement subject suffering from overactive bladder or the like.

[Citation List]

[Patent Literature]

[PTL 1] Japanese Patent No. 4677615
[PTL 2] Japanese Unexamined Patent Application Publication No. 2009-512532
[PTL 3] Japanese Unexamined Patent Application Publication No. 2000-210286
[PTL 4] US Patent No. 6565512
[PTL 5] Japanese Unexamined Patent Application Publication No. 2007-508857

[PTL 6] Japanese Unexamined Patent Application Publication No. 2011-183142
[PTL 7] Japanese Unexamined Patent Application Publication No. 2014-23813

[Summary of Invention]

[Technical Problem]

The present invention provides an ultrasonic urine volume measuring instrument, and urine volume management data generating method using ultrasonic urine volume measuring instrument that can highly accurately measure urine volume in the bladder and its temporal changes as needed and that can reduce power consumption.

[Solution to Problem]

In order to solve the above-described problem, an ultrasonic urine volume measuring instrument according to the present invention includes: an ultrasound probe that includes an ultrasound device that transmits ultrasound toward bladder of a measurement subject at a certain measurement cycle and receives a wave reflected from a wall of the bladder; an ultrasound controller that controls transmission and reception of ultrasound and its reflected wave, the transmission and reception being performed by the ultrasound device; and an arithmetic unit that calculates, on the basis of the reflected wave, urine volume in the bladder said every measurement cycle. The arithmetic unit includes a comparator that compares the calculated urine volume in the bladder with a certain first threshold. The measurement cycle of ultrasound transmitted from the ultrasound device is longer in a case where the urine volume in the bladder compared by the comparator is less than the first threshold than that in a case where the urine volume is greater than or equal to the first threshold. The ultrasonic urine volume measuring instrument further includes a timer unit capable of causing, in a case where the urine volume in the bladder compared by the comparator is less than the first threshold, the arithmetic unit to enter a sleep state during each measurement cycle in order to reduce power consumption, and causing the arithmetic unit to recover every measurement cycle.

In addition, an ultrasonic urine volume measuring instrument according to the present invention includes: an ultrasound probe that includes an ultrasound device that transmits ultrasound toward bladder of a measurement subject at a certain measurement cycle and receives a wave reflected from a wall of the bladder; an ultrasound controller that controls transmission and reception of ultrasound and its reflected wave, the transmission and reception being performed by the ultrasound device; and an arithmetic unit that calculates, on the basis of the reflected wave, urine volume in the bladder said every measurement cycle. The ultrasonic urine volume measuring instrument further includes a mode selector capable of selecting one of a measurement mode of measuring urine volume while the ultrasound probe is placed at a certain measurement position on stomach of the measurement subject, and a positioning mode of measuring urine volume while moving the ultrasound probe to a plurality of tentative measurement positions on the stomach in order to determine the measurement position suitable for placing the ultrasound probe in the measurement mode. The arithmetic unit includes a comparator that compares the calculated urine volume in the bladder with a certain first threshold. The ultrasound controller is capable of: causing, in the positioning mode, the ultrasound device to transmit ultrasound at a certain first cycle; causing, in the measurement mode, the ultrasound device to transmit ultrasound at a certain second cycle in a case where the urine volume in the bladder compared by the comparator is greater than or equal to the first threshold; and causing, in the measurement mode, the ultrasound device to transmit ultrasound at a third cycle longer than the first cycle and the second cycle in a case where the urine volume in the bladder compared by the comparator is less than the first threshold.

In addition, an ultrasonic urine volume measuring instrument according to the present invention includes: an ultrasound probe that includes an ultrasound device that transmits ultrasound toward bladder of a measurement subject at a certain measurement cycle and receives a wave reflected from a wall of the bladder; an ultrasound controller that controls transmission and reception of ultrasound and its reflected wave, the transmission and reception being performed by the ultrasound device; and an arithmetic unit that calculates, on the basis of the reflected wave, urine volume in the bladder said every measurement cycle. The ultrasonic urine volume measuring instrument further includes a mode selector capable of selecting one of a measurement mode of measuring urine volume while the ultrasound probe is placed at a certain measurement position on stomach of the measurement subject, and a positioning mode of measuring urine volume while moving the ultrasound probe to a plurality of tentative measurement positions on the stomach in order to determine the measurement position suitable for placing the ultrasound probe in the measurement mode. The arithmetic unit includes a comparator that compares the calculated urine volume in the bladder with a certain first threshold. The arithmetic unit is capable of calculating, in the positioning mode, urine volume in the bladder on the basis of a first number of reflected waves chronologically received at a certain first cycle; measuring, in the measurement mode, in a case where the urine volume in the bladder compared by the comparator is greater than or equal to the first threshold, urine volume in the bladder on the basis of a certain second number of reflected waves chronologically received at a certain second cycle, the second number being greater than the first number; and measuring, in the measurement mode, in a case where the urine volume in the bladder compared by the comparator is less than the first threshold, urine volume in the bladder on the basis of a plurality of reflected waves chronologically received at a certain third cycle that is longer than the first cycle and the second cycle.

In this case, it is preferable that the first cycle and the second cycle be the same cycle.

In addition, the comparator may further compares the calculated urine volume in the bladder with a certain second threshold that is greater than the first threshold, and the ultrasonic urine volume measuring instrument may further include a reporting unit that reports, in a case where the urine volume in the bladder compared by the comparator is greater than or equal to the second threshold, that the urine volume is greater than or equal to the second threshold.

In addition, it is preferable that the ultrasonic urine volume measuring instrument according to the present invention further include: a micturition input unit used by the measurement subject to input a micturition signal at a time the measurement subject feels micturition; a measurement data memory that stores the urine volume in the bladder calculated by the arithmetic unit, its measurement time, and a micturition time corresponding to an input time of the micturition signal; a maximum urine volume memory that stores maximum urine volume in the bladder that is a maximum value among urine volumes in the bladder calculated by the arithmetic unit in a certain period; and a display unit that displays, at the time of inputting the micturition signal, retained urine volume upon micturition that is retained urine volume corresponding to the micturition time, and the maximum urine volume in the bladder.

In this case, the retained urine volume upon micturition may be urine volume in the bladder calculated by the arithmetic unit in a certain time prior to the time of inputting the micturition signal.

Alternatively, the retained urine volume upon micturition may be urine volume in the bladder newly calculated by the arithmetic unit after the ultrasound device transmits ultrasound at the time of inputting the micturition signal.

In addition, the ultrasonic urine volume measuring instrument may further include an urination input unit used by the measurement subject to input a urination signal when the measurement subject urinates, and the measurement data memory may further store a urination time corresponding to an input time of the urination signal input using the urination input unit.

Furthermore, the display unit may display urine volume in the bladder used in at least one of toilet training and bladder training.

In addition, a urine volume management data generating and displaying method using the ultrasonic urine volume measuring instrument according to the present invention includes: a step of generating, on the basis of urine volume in the bladder and its measurement time, and the micturition time, stored in the ultrasonic urine volume measuring instrument, urine volume management data by chronologically arranging at least urine-volume-in-bladder data including the urine volume in the bladder and its measurement time, and micturition data including the micturition time; and a step of displaying at least part of the urine volume management data in the form of a table or a graph.

In this case, the urine volume management data may further include urination time data corresponding to an input time of a urination signal input by the measurement subject when the measurement subject urinates.

In that case, it is preferable that the method further include: a step of calculating the number of micturitions that is the number of micturition signals in a certain period on the basis of the micturition data, and the number of urinations that is the number of urination start signals or urination end signals in a certain period on the basis of the urination time data; and a step of displaying the maximum urine volume in the bladder, the number of micturitions, and the number of urinations.

It is also preferable that the urine volume management data generating and displaying method further include a step of searching the urine-volume-in-bladder data for a urine volume decrease time at which urine volume in the bladder decreases with time, and storing the amount of decrease as urination volume. It is preferable that the urine volume management data include the urination volume.

In that case, it is preferable that the method further include a step of calculating a time difference between each urine volume decrease time and the urination time or the micturition time. It is preferable that the urine volume management data include the time difference or data calculated from the time difference.

[Advantageous Effects of Invention]

According to the present invention, the measurement cycle of ultrasound to be transmitted becomes a longer cycle in the case where the calculated urine volume in the bladder is less than a first threshold than that in the case where the urine volume is greater than or equal to the first threshold, and there is provided a timer unit capable of causing, in the case where the calculated urine volume in the bladder is less than the first threshold, an arithmetic unit to enter a sleep state during each measurement cycle in order to reduce power consumption, and causing the arithmetic unit to recover every measurement cycle. Accordingly, the urine volume in the bladder can be highly accurately measured when the urine volume is greater than or equal to the first threshold, and power consumption can be reduced when the urine volume is less than the first threshold.

[Brief Description of Drawings]

[Fig. 1] Fig. 1 is a schematic block diagram according to an embodiment of the present invention.
[Fig. 2] Fig. 2 is a schematic diagram illustrating a state in which an ultrasound device is attached to a measurement subject.
[Fig. 3] Fig. 3 is a schematic diagram illustrating a screen and mode selecting buttons of an ultrasonic urine volume measuring instrument.
[Fig. 4] Fig. 4 is a schematic diagram of the waveform of a reflected wave received by each ultrasound device.
[Fig. 5] Fig. 5 is a time chart for describing a transmission cycle and a measurement cycle of each ultrasound device.
[Fig. 6] Fig. 6 is a schematic block diagram that specifically illustrates Fig. 1 in order to describe a positioning mode.
[Fig. 7] Fig. 7 is a schematic diagram illustrating a display example of a display unit in the positioning mode and the mode selecting buttons.
[Fig. 8] Fig. 8 is a time chart for describing a retained urine volume value based on five ultrasound reflected waves.
[Fig. 9] Fig. 9 is a flowchart describing the positioning mode.
[Fig. 10] Fig. 10 is a time chart for describing a retained urine volume value based on ten ultrasound reflected waves.
[Fig. 11] Fig. 11 is a schematic diagram illustrating a display example of the display unit in an alarm mode.
[Fig. 12] Fig. 12 is a schematic block diagram that specifically illustrates Fig. 1 in order to describe a low power consumption mode.
[Fig. 13] Fig. 13 is a flowchart describing the low power consumption mode.
[Fig. 14] Fig. 14 is a schematic block diagram that specifically illustrates a timer unit for causing an arithmetic unit in Fig. 1 to enter a sleep state.
[Fig. 15] Fig. 15 is a schematic block diagram illustrating an example of a urine volume measurement system that generates urine volume management data.
[Fig. 16] Fig. 16 is an elevation of a measuring instrument main body in which various types of data including retained urine volume measured by the system of Fig. 15 are displayed on a screen.
[Fig. 17] Fig. 17 represents, in the form of a table, retained urine at each time, stored in a measurement data memory.
[Fig. 18] Fig. 18 represents, in the form of a table, a micturition time stored in the measurement data memory.
[Fig. 19A and Fig. 19B] Fig. 19A and Fig. 19B are display examples displaying, on the display unit, a retained urine volume value upon micturition in response to an input of a micturition signal to a micturition input unit.
[Fig. 20] Fig. 20 is a first display example representing, in the form of a table, urine volume management data.
[Fig. 21] Fig. 21 is a second display example representing, in the form of a table, urine volume management data.
[Fig. 22] Fig. 22 represents, as a graph, urine volume management data.
[Fig. 23] Fig. 23 is a third display example representing, in the form of a table, urine volume management data.
[Fig. 24] Fig. 24 is a fourth display example representing, in the form of a table, urine volume management data.
[Fig. 25] Fig. 25 is a schematic block diagram illustrating another example of the urine volume measurement system of Fig. 15.
[Fig. 26] Fig. 26 is a fifth display example representing, in the form of a table, urine volume management data.

[Description of Embodiments]

<Embodiment of Basic Configuration>

Hereinafter, the configuration of an ultrasonic urine volume measuring instrument according to an embodiment of the present invention will be described on the basis of Figs. 1, 2, and 3. As illustrated in Fig. 1, an ultrasonic urine volume measuring instrument 1 mainly includes an ultrasound probe 10 and a controller 20.

As illustrated in Fig. 2, a plurality of (four in the diagram) ultrasound devices 11 are arranged in a straight line and provided in the ultrasound probe 10 so as to transmit ultrasound in the shape of a fan that opens in a vertical direction with respect to the bladder, and to receive waves reflected from the bladder. Specifically, the ultrasound probe 10 is fixed via an ultrasound transmission medium such as ultrasound gel so that the lower end of the ultrasound probe 10 is positioned on a stomach surface corresponding to the upper end of the pubis, and the plurality of ultrasound devices 11 are arranged along the vertical direction of the bladder.

As illustrated in Fig. 1, the controller 20 roughly includes an ultrasound controller 21 that controls transmission and reception, by the ultrasound devices 11, of ultrasound and its reflected waves, an input unit 22 used by a measurement subject or a health professional such as a doctor to input data, a memory 23 that stores various types of data regarding urine volume, an arithmetic unit 24 that performs various arithmetic operations regarding urine volume on the basis of, for example, reflected wave data from the ultrasound controller 21, an output unit 25 that outputs arithmetic data obtained by the arithmetic unit 24, input data, and the like, an alarm unit 26 serving as a reporting unit that produces an alarm on the basis of the arithmetic data and input data, and a display unit 27 for displaying the arithmetic data, the input data, and the like. The arithmetic unit 24 also plays a role of controlling other elements in the controller 20, and is configured by a CPU and the like.

Besides controlling transmission, by the ultrasound devices 11, of ultrasound, the ultrasound controller 21 also performs reception control of amplifying reflected waves received by the ultrasound devices 11 and converting the amplified waves to digital signals, thereby obtaining reflected wave data. The reflected wave data is stored in the memory 23.

The input unit 22 is used to input, as input data, a signal regarding mode selection, which will be described later, and other data. The input data is stored in the memory 23.

The arithmetic unit 24 has a retained urine volume calculator 24a that calculates urine volume in the bladder every measurement cycle on the basis of the reflected wave data. The urine volume calculated by the retained urine volume calculator 24a is stored in the memory 23.

The display unit 27 chronologically displays, in the form of a graph, urine volume calculated by the retained urine volume calculator 24a, and various types of data regarding urine volume measurement, and specifically a screen of the ultrasonic urine volume measuring instrument 1 such as that illustrated in Fig. 3 corresponds to the display unit 27. A specific example of display in the form of a graph on the screen includes display of a bar graph presenting a plurality of urine volumes measured within a certain time range that are arranged in a time axis direction in which time is plotted on the horizontal axis and urine volume is plotted on the vertical axis. In addition, the numeral 186 on the screen represents the most recent measured urine volume. Note that the urine volume in the bladder may be displayed in the form of, for example, a line graph.

The output unit 25 outputs various types of data, such as arithmetic data including the urine volume in the bladder calculated by the retained urine volume calculator 24a, and input data input using the input unit 22, to an external device connected to the ultrasonic urine volume measuring instrument 1. At this time, connection between the ultrasonic urine volume measuring instrument 1 and the external device may be wired or wireless.

Next, on the basis of Figs. 4 and 5, a method of calculating urine volume in the bladder, which is used in the embodiment, will be described.

First, ultrasound transmitted by the ultrasound devices 11 is reflected at the boundary between tissues. Therefore, ultrasound transmitted by the ultrasound devices 11 toward the bladder is reflected at the front wall and the back wall of the bladder, and these reflected waves are received by the ultrasound devices 11.

Fig. 4 is a schematic diagram of the waveform of a reflected wave received by each ultrasound device 11. The schematic diagram is represented as a diagram in which reflection intensity is plotted on the vertical axis and time (or distance) since transmission of ultrasound is plotted on the horizontal axis. Here, let Pi represent the peak intensity of a reflected wave from the back wall, out of the waveform received by the i-th ultrasound device 11, and let Di represent a peak-to-peak distance between peaks of intensity of reflected waves from the front wall and the back wall, urine volume EU retained in the bladder can be calculated on the basis of the following equations (1) and (2).

$$\mathrm{PDj} = \sum \mathrm{Pi} \times \mathrm{Di} \qquad ..(1)$$

$$\mathrm{EU} = \mathrm{PD} \times \mathrm{R} \qquad ..(2)$$

Here, Pdj represents a measurement index value, EU represents the calculated urine volume in the bladder, PD represents an average index value described later, and R represents a coefficient defined in accordance with differences among individuals on the basis of the anatomical structure, and posture during measurement. In addition, j in PDj means the position in a series of ultrasound waves transmitted every certain transmission cycle described later, and in this case j is an integer from 1 to 10. Also, i in Pi and Di means a number given to each of the plurality of ultrasound devices 11 included in the ultrasound probe 10, and in this case i is an integer from 1 to 4.

In the ultrasonic urine volume measuring instrument 1 of the embodiment, because the measurement is performed in ultrasound A mode, measurement results are susceptible to noise. Therefore, in measurement of urine volume in the bladder, it is desirable to use ultrasound reflected waves obtained at a plurality of times, and to eliminate noise by statistical processing. Specifically, each of the ultrasound devices 11 transmits ultrasound every certain transmission cycle, such as once every 0.1 seconds, for ten times (one second). Each measurement index value PDj in each transmission cycle can be obtained by adding the product of the reflection intensity Pi and the peak-to-peak distance Di of reflected waves received by each ultrasound device 11 for i = 1 to 4, and, as a result, ten measurement index values PDj can be obtained in one measurement. From these measurement index values PDj (j = 1 to 10), the maximum value and the minimum value, which may be noise, are eliminated, and furthermore appropriate averaging is performed, thereby obtaining an average index value PD. By multiplying the average index value PD by the coefficient R, urine volume EU in the bladder in each measurement cycle can be calculated. The urine volume EU calculated in this manner is chronologically stored, along with its measurement time, in the memory 23.

The above-mentioned ultrasonic urine volume measuring instrument 1 of the embodiment performs the following operations on the basis of an input from the outside to the input unit 22.

(A) Positioning of ultrasound device (positioning mode)
(B) Residual urine measurement (residual urine mode)
(C) Regular measurement (regular mode)
(D) Urine volume report measurement (alarm mode)
(E) Low power consumption urine volume report measurement (low power consumption mode)
(F) Generation/recording of urine volume management data Hereinafter, these operations will be described. Note that the above-mentioned (B) to (E) will be collectively referred to as measurement modes in the following description.

(A) Positioning of ultrasound device (positioning mode)

In the ultrasonic urine volume measuring instrument 1, as has been described above, at the time of measurement, it is necessary to place the ultrasound probe 10 such that the lower end thereof will be positioned on a stomach surface corresponding to the upper end of the pubis, as illustrated in Fig. 2. A measurement value obtained by the ultrasound probe 10 sensitively reacts to the position and angle at which the ultrasound probe 10 is placed. Therefore, in order to more accurately measure the urine volume, it is necessary to highly precisely position the ultrasound probe 10 at a position and an angle suitable for measurement. The positioning is not always easy, and some experience is necessary. To this end, in the ultrasonic urine volume measuring instrument 1 according to the embodiment, while the ultrasound probe 10 is placed at a certain measurement position on the stomach of a measurement subject, it is possible to selectively switch between the above-mentioned measurement modes ((B), (C), (D), and (E) operations, which will be described later) of measuring urine volume retained in the bladder every certain measurement cycle, and the positioning mode of measuring the urine volume while moving the ultrasound probe 10 to a plurality of tentative measurement positions on the stomach in order to determine the measurement position suitable for placing the ultrasound probe 10 in the measurement modes. Here, the tentative measurement positions are positions at which the ultrasound probe 10 is temporarily placed in order to find a position at which urine volume in the bladder can be measured with high accuracy.

Hereinafter, the positioning mode will be specifically described.

Fig. 6 is a diagram that specifically illustrates the block diagram of Fig. 1 in order to describe the positioning mode. As illustrated in Fig. 6, the controller 20 roughly includes the ultrasound controller 21, the input unit 22, the memory 23, the arithmetic unit 24, the output unit 25, the alarm unit 26, and the display unit 27, and the schematic configurations thereof are as described in

<Embodiment of Basic Configuration>. Thus, overlapping descriptions are omitted except when necessary.

The display unit 27 chronologically displays, in the form of a graph, urine volume calculated by the retained urine volume calculator 24a, and various types of data regarding urine volume measurement, and specifically a screen of the ultrasonic urine volume measuring instrument 1 illustrated in Fig. 7 corresponds to the display unit 27. A specific example of display in the form of a graph on the screen includes display of a bar graph presenting a plurality of urine volumes measured within a certain time range that are arranged in a time axis direction in which time is plotted on the horizontal axis and urine volume is plotted on the vertical axis. In addition, the numeral 186 on the screen represents the most recent measured urine volume, and the numeral 243 represents the maximum sensitivity value serving as a positioning index value, which will be described later (the maximum urine volume value at the time of positioning, hereinafter referred to as the "maximum value"). Note that the urine volume in the bladder may be displayed in the form of, for example, a line graph.

The input unit 22 has a mode selector 22a that switches the mode among the above-mentioned measurement modes and the positioning mode by pressing any one button included in a mode selecting button group 43. Specifically, the mode selector 22a switches the mode to the positioning mode in response to pressing a predetermined button included in the mode selecting button group 43 while one of the measurement modes is being selected, and the mode selector 22a switches the mode to any one of the various measurement modes in response to pressing a corresponding button included in the mode selecting button group 43 while the positioning mode is being selected.

Since it is desirable to conduct positioning at the start of each measurement, switching the mode to the positioning mode may be done in response to pressing a power on button (not illustrated). In this case, the mode is always switched to the positioning mode at the time power is turned on, and positioning is conducted.

Here, the ultrasound controller 21 may control the ultrasound devices 11 so that the measurement cycle in the various measurement modes becomes a first measurement cycle, such as one minute, and the measurement cycle in the positioning mode becomes a second measurement cycle shorter than the first measurement mode, such as one second (see Fig. 5). Needless to say, the first measurement cycle and the second measurement cycle may be the same. In addition, although the number of times ultrasound is transmitted in one measurement is ten times, as illustrated in Fig. 5, the number is not limited to ten times. In addition, the number of times ultrasound is transmitted may be changed from one measurement to another in accordance with, for example, the body movement of the measurement subject at the time of measurement.

As has been described above, calculation of urine volume in the bladder is performed by statistically processing data based on ultrasound reflected waves (a first number of reflected waves) at a plurality of times in the past and at present. In the example of the embodiment, urine volume in the bladder is calculated on the basis of ultrasound reflected waves at ten consecutive times in the various measurement modes. In other words, the value of urine volume in the bladder can only be calculated after ten reflected waves are obtained. Thus, a little time lag occurs until the value of urine volume in the bladder is calculated. In addition, since many ultrasound reflected waves are used, the reactivity of the calculated urine volume in the bladder is inferior with respect to changes in the position of the ultrasound probe 10. To this end, the statistical processing method may be changed so that the number of reflected waves used in urine volume measurement is reduced from the first number to a second number in the positioning mode, such as the case where urine volume in the bladder is calculated on the basis of five ultrasound reflected waves, instead of ten ultrasound reflected waves in a normal measurement mode. In this case, a time until urine volume in the bladder is first output after entering the positioning mode becomes shorter than a time until urine volume in the bladder is first output after entering a normal measurement mode, and the reactivity to changes in the position of the ultrasound probe 10 becomes higher.

Furthermore, as illustrated in Fig. 8, the same ultrasound reflected waves may be overlappingly used in calculating a plurality of values of urine volume in the bladder. In other words, in the case of calculating urine volume in the bladder on the basis of five ultrasound reflected waves, urine volume M1 may calculated at time T5 on the basis of ultrasound reflected waves U1 to U5, and urine volume M2 may be calculated at time T6 on the basis of ultrasound reflected waves U2 to U6. In this case, the urine volume calculation interval can be shortened to the ultrasound transmission interval. For example, in the example where the ultrasound transmission interval is 0.1 seconds, the value of urine volume in the bladder can be consecutively obtained at an interval of 0.1 seconds.

As illustrated in Fig. 6, the memory 23 has an index value memory 23a that stores the maximum value among urine volumes measured at a plurality of tentative measurement positions while the positioning mode is being selected. In addition, the arithmetic unit 24 has a first comparator 24c that compares urine volume measured while the positioning mode is being selected, with a positioning index value indicating an appropriate value for positioning the ultrasound probe 10. In the case where the calculated urine volume value is greater than or equal to the maximum value, the maximum value stored in the index value memory 23a is updated to the calculated urine volume value. In addition, in the case where the calculated urine volume value is less than the maximum value, the maximum value stored in the index value memory 23a is maintained. Note that the maximum value stored in the index value memory 23a may be updated in the case where the calculated urine volume value is greater than the maximum value.

In addition, the index value memory 23a may be configured to additionally store an allowance value meaning allowable sensitivity with respect to the maximum value. The allowance value may be set within a range not exceeding the maximum value, and the allowance value may be automatically set by multiplying the maximum value by a certain ratio or may be manually input from the input unit 22. In this case, the automatic setting of the allowance value is performed by an allowance value calculator 24b included in the arithmetic unit 24.

As illustrated in Fig. 7, on the bar graph displayed on the screen included in the display unit 27, the maximum value is displayed as a first positioning index value 41 using a straight line extending in the time axis direction. In the case of using the allowance value, the allowance value is displayed as a second positioning index value 42 using a straight line extending in the time axis direction. The display unit 27 can change the display scale of the graph in accordance with the maximum value. For example, the greater the maximum value becomes as a result of being updated, the greater the maximum value of the urine volume axis of the bar graph becomes. In addition, it is only necessary that at least one of the first positioning index value 41 and the second positioning index value 42 be displayed on the bar graph, and both the first positioning index value 41 and the second positioning index value 42 need not be displayed.

The alarm unit 26 notifies the measurement subject or the like by producing an alarm in the case where the urine volume calculated in the positioning mode reaches or becomes greater than the first positioning index value 41 or the second positioning index value 42. There are several types of alarm including sound, a screen, and vibration, but the type of alarm is not limited thereto. In addition, an alarm used for the first positioning index value 41 and an alarm used for the second positioning index value 42 may be different. Furthermore, the alarm unit 26 may produce an acoustic signal or vibration at an interval in accordance with the reciprocal of the difference between the calculated urine volume and the first positioning index value 41 or the second positioning index value 42. For example, in the case where the calculated urine volume is close to the first positioning index value 41 or the second positioning index value 42, the alarm unit 26 may produce vibration or acoustics at a short producing interval. In contrast, in the case where the difference between the calculated urine volume and the first positioning index value 41 or the second positioning index value 42 is great, the alarm unit 26 may produce an acoustic signal or vibration at a long producing interval. This can be implemented by the arithmetic unit 24 which obtains the difference between the calculated urine volume and the first positioning index value 41 or the second positioning index value 42 and determines an alarm interval by multiplying the difference by a coefficient, or which compares the difference with a plurality of thresholds and stepwisely determines an alarm interval on the basis of the comparison results.

Next, on the basis of Fig. 9, a method of positioning the ultrasound probe 10 using the ultrasonic urine volume measuring instrument 1 will be described. In the positioning method described hereinafter, an example in which the allowance value (second positioning index value 42) is not set will be described.

First of all, the mode is changed from one of the measurement modes to the positioning mode in response to pressing a corresponding button included in the mode selecting button group 43 attached to the ultrasonic urine volume measuring instrument 1 (step S10). Step S10 here corresponds to a first step of the present invention. Next, the maximum value stored in the index value memory 23a is reset (step S11). Next, the retained urine volume calculator 24a measures urine volume in the bladder every second measurement cycle (step S12). The measured urine volume is displayed as a numeral and a bar graph on the display unit 27 (step S13).

Next, the measured urine volume is compared with the maximum value by the first comparator 24c (step S14). Step S14 here corresponds to a fourth step of the present invention. In the case where the measured urine volume is greater than or equal to the maximum value, the measured urine volume is updated as the maximum value by the first comparator 24c (step S15). Step S15 here corresponds to a second step of the present invention. In contrast, in the case where the measured urine volume is less than the maximum value, above-described step S15 and later-described step S16 to S18 are not performed, and it is determined whether to cancel the positioning mode (step S19).

Next to step S15, the positioning index value 41 displayed on the bar graph on the screen is updated in accordance with the updated maximum value (step S16), and the scale of the bar graph is changed in accordance with the updated positioning index value 41 (step S17). By producing an alarm, the measurement subject or the like is notified that the positioning index value 41 has been updated (step S18). Here, step S17 corresponds to a third step of the present invention, and step S18 corresponds to a fifth step of the present invention.

Next, in step S19 described above, it is determined whether the cancellation of the positioning mode is being executed. In the case where the cancellation of the positioning mode is not being executed, the operation returns to step S12. Therefore, steps S12 to S19 described above are repeatedly executed until the positioning mode ends. Step S19 here corresponds to the first step of the present invention. While steps S12 to S19 are repeatedly executed, after a position and angle at which the ultrasound probe 10 is placed that are suitable for urine volume measurement in the measurement modes are checked on the basis of the maximum value, the positioning mode is canceled. The cancellation of the positioning mode is performed in response to pressing a button included in the mode selecting button group 43, and accordingly the mode is switched to one of the measurement modes (step S20). When the mode is switched to one of the various measurement modes, the method of positioning the ultrasound probe 10 ends. Once the position and angle at which the ultrasound probe 10 is placed are determined, the ultrasound probe 10 is fixed to the stomach in that state. The ultrasound probe 10 can be fixed by using an adhesive tape or gel.

According to the positioning mode of the embodiment, the maximum urine volume value among urine volume values measured at tentative measurement positions while the positioning mode is being selected, that is, the maximum value, and/or the allowance value is displayed as a positioning index value for positioning the ultrasound probe 10, on a graph of the urine volume values measured in the positioning mode on the display unit 27, and positioning is supported by an alarm. Therefore, it is possible to easily position, on the basis of the index value, the ultrasound probe 10 at a position and angle at which the ultrasound probe 10 is placed that are suitable for urine volume measurement in the various measurement modes.

(B) Residual urine measurement (residual urine measurement mode)

Next, residual urine measurement will be described. In residual urine measurement, urine volume retained in the bladder at a measurement time point is measured.

The residual urine measurement is preferably conducted in a state where the position of the ultrasound probe 10 is determined in the above-mentioned positioning mode. In this case, for example, when the position of the ultrasound probe 10 is determined in the positioning mode, pressing a button for residual urine measurement included in the mode selecting button group 43 ends the positioning mode and starts a residual urine measurement mode.

In the residual urine measurement, each ultrasound device 11 in the ultrasound probe 10 transmits ultrasound. Each ultrasound device 11 receives its reflected waves, and the arithmetic unit 24 uses equations (1) and (2) discussed in <Embodiment of Basic Configuration> to calculate urine volume in the bladder. Specifically, for example, each ultrasound device 11 transmits ultrasound ten times at an interval of 0.1 seconds, and obtains ten items of reflected wave data. Using the ten items of reflected wave data, the arithmetic unit 24 calculates urine volume in the bladder, from which noise has been eliminated by a statistical processing technique.

The display unit 27 obtains the calculated urine volume in the bladder from the arithmetic unit 24, and displays its numeral.

(C) Regular measurement (regular measurement mode)

The regular measurement is preferably conducted in a state where the position of the ultrasound probe 10 is fixed in the above-mentioned positioning mode. In this case, for example, when the position of the ultrasound probe 10 is determined in the positioning mode, pressing a button for regular measurement included in the mode selecting button group 43 ends the positioning mode and starts a regular measurement mode.

In the regular measurement mode, instead of measuring urine volume in the bladder only once like the residual urine measurement, urine volume is consecutively calculated for a certain period, and calculation results are displayed using numerals and/or a graph. For example, urine volume in the bladder is continuously measured at a certain interval, that is, once every minute, and the measurement results are displayed in a graph and are stored in association with measurement times. That is, the retained urine volume calculator 24a calculates urine volume in the bladder every certain measurement cycle, such as once every minute. At this time, each ultrasound device 11 transmits ultrasound every certain transmission cycle, such as once every 0.1 seconds, for ten times (one second), in order to calculate urine volume in this measurement cycle. Therefore, each measurement index value PDj in each transmission cycle can be obtained by adding the product of the reflection intensity Pi and the peak-to-peak distance Di of the reflected waves received by each ultrasound device 11 for i = 1 to 4, and, as a result, ten measurement index values PDj (see equations (1) and (2) above) can be obtained in one measurement cycle. By performing appropriate averaging of these measurement index values PDj (j = 1 to 10), an average index value PD can be obtained, which is then multiplied by the coefficient R to calculate urine volume EU in the bladder in each measurement cycle. The urine volume EU calculated in this manner is chronologically stored, along with its measurement time, in the memory 23. The display unit 27 displays a graph and numerals, as illustrated in Fig. 3.

Although it has been described here that the measurement cycle is one minute, if ultrasound is transmitted every 0.1 seconds, the measurement cycle can be set to 0.1 seconds at the shortest. Fig. 10 is a diagram describing a measurement interval in this case. As illustrated in Fig. 10, urine volume value N1 in the bladder calculated at first at time T10 since the start of the regular measurement mode is obtained from ultrasound reflected waves U1 to U10 based on ultrasound transmission, and urine volume value N2 in the bladder calculated next at time T11 is obtained from ultrasound reflected waves U2 to U11 based on ultrasound transmission. By calculating the values of urine volume in the bladder using the same ultrasound reflected waves as described above, the interval of measuring the value of urine volume in the bladder can be reduced to the ultrasound transmission interval.

(D) Urine volume report measurement (alarm mode)

The alarm mode is preferably conducted in a state where the position of the ultrasound device 11 is fixed in the above-mentioned positioning mode. In this case, for example, when the position of the ultrasound device 11 is determined in the positioning mode, pressing a button for the alarm mode included in the mode selecting button group 43 ends the positioning mode and starts the alarm mode.

In the alarm mode, like the regular measurement, urine volume in the bladder is consecutively calculated. For example, urine volume in the bladder is calculated at a certain interval, such as once every minute. An alarm is produced in the case where the calculated urine volume in the bladder becomes greater than or equal to a second threshold (alarm urine volume), thereby notifying the measurement subject.

That is, referring to Fig. 1, on the basis of a signal from the ultrasound controller 21, each ultrasound device 11 transmits ultrasound at, for example, a certain interval. Each ultrasound device 11 receives its reflected waves, and the arithmetic unit 24 calculates urine volume in the bladder on the basis of the reflected waves. The calculation method is the same as that in the above-mentioned regular measurement mode. In the alarm mode, the arithmetic unit 24 compares the calculated urine volume in the bladder with the second threshold (alarm urine volume) and, when the urine volume in the bladder becomes greater than or equal to the alarm urine volume, an alarm is produced. There are several types of alarm including sound, a screen, and vibration, but the type of alarm is not limited thereto. An alarm may be any type as long as it can notify the measurement subject or the like.

In the alarm mode, the display unit 27 displays various types of data regarding urine volume measurement, such as the alarm urine volume and urine volume in the bladder, and chronologically displays the aforementioned urine volume in a graph. Specifically, a screen of the ultrasonic urine volume measuring instrument 1 such as that illustrated in Fig. 11 corresponds to the display unit 27. Specific examples of data displayed on the screen include display of A: 350 indicating the alarm urine volume, N: 60 indicating a later-described first time interval, the numeral 46 indicating the most recent urine volume in the bladder, and PA: 10 indicating a later-described second time interval. In addition, a specific example of display in the form of a graph on the screen includes display of a bar graph presenting a plurality of urine volumes that are arranged in a time axis direction in which time is plotted on the horizontal axis and urine volume is plotted on the vertical axis. Note that the bar graph may be, for example, a line graph.

The alarm urine volume is generally urine volume determined that the measurement subject is supposed to urinate, and the alarm urine volume is set in advance on the basis of the size of the bladder and lifestyle of each measurement subject. Specifically, measurements in the above-mentioned regular mode are executed for a few days, and changes in urine volume in the bladder are observed, thereby determining the urine volume to be urinated. In another embodiment, the maximum urine volume in the bladder may be obtained from log data of measurement in the regular mode, and, on the basis of the maximum urine volume in the bladder, the alarm urine volume may be automatically set to, for example, 95% of the maximum urine volume in the bladder.

According to the alarm mode, it becomes possible to notify a patient who has difficulty in sensing micturition, such as a patient with overactive bladder, that the bladder is filled with urine at an appropriate timing.

(E) Low power consumption urine volume report measurement (low power consumption mode)

In the ultrasonic urine volume measuring instrument 1 of the embodiment, the controller 20 and/or the ultrasound probe 10 in Fig. 1 is driven by a battery. Since measurement is conducted continuously for a long period of time in the above-described regular mode and alarm mode, it is desirable to reduce power consumption. In the low power consumption mode, the above-described alarm mode is executed while reducing power consumption.

The low power consumption mode is conducted in a state where the position of the ultrasound probe 10 is fixed after going through the above-mentioned positioning mode. For example, pressing a button for starting the positioning mode, included in the button group 43, or turning on power starts the positioning mode, as described above. After the positioning mode is completed, selecting the low power consumption mode ends the positioning mode and starts the low power consumption mode. In the embodiment, pressing one button included in the button group 43 twice selects the low power consumption mode.

Hereinafter, the low power consumption mode will be specifically described.

Fig. 12 is a block diagram that specifically illustrates the block diagram of Fig. 1 in order to describe the low power consumption mode. As illustrated in Fig. 12, the controller 20 roughly includes the ultrasound controller 21, the input unit 22, the memory 23, the arithmetic unit 24, the output unit 25, the alarm unit 26, and the display unit 27, and the schematic configurations thereof are as described in <Embodiment of Basic Configuration>. Thus, overlapping descriptions are omitted except when necessary.

The arithmetic unit 24 includes a second comparator 24d that compares urine volume in the bladder with a certain first threshold and a certain second threshold, which will be described later, and the retained urine volume calculator 24a, which calculates urine volume in the bladder on the basis of the reflected wave data. The urine volume in the bladder, calculated by the retained urine volume calculator 24a, is stored in the memory 23.

The alarm unit 26 compares, with the use of the second comparator 24d, the urine volume in the bladder calculated by the retained urine volume calculator 24a with the alarm urine volume, which means the urine volume prompting the measurement subject to urinate, and, in the case where the urine volume in the bladder is greater than or equal to the alarm urine volume, produces an alarm. The alarm urine volume corresponds to the second threshold. There are several types of alarm including sound, a screen, and vibration, but the type of alarm is not limited thereto. An alarm may be any type as long as it can notify the measurement subject or the like.

The display unit 27 displays various types of data regarding urine volume measurement, such as the alarm urine volume and urine volume in the bladder, and chronologically displays the aforementioned urine volume in a graph. Specifically, a screen of the ultrasonic urine volume measuring instrument 1 such as that illustrated in Fig. 11 corresponds to the display unit 27. Specific examples of data displayed on the screen include display of A: 350 indicating the alarm urine volume, N: 60 indicating a later-described first time interval, the numeral 46 indicating the most recent urine volume in the bladder, and PA: 10 indicating a later-described second time interval. In addition, a specific example of display in the form of a graph on the screen includes display of a bar graph presenting a plurality of urine volumes that are arranged in a time axis direction in which time is plotted on the horizontal axis and urine volume is plotted on the vertical axis. Note that the bar graph may be, for example, a line graph.

Regarding the method of calculating urine volume retained in the bladder, the calculation method of (1) and (2) discussed in <Embodiment of Basic Configuration> is used. Using this calculation method, urine volume EU in the bladder is calculated every measurement cycle. The calculated urine volume EU is chronologically stored in the memory 23.

In order for the ultrasonic urine volume measuring instrument 1 to obtain, on the basis of the above calculation method, more accurate urine volume in the bladder and its temporal changes, it is desirable to conduct measurement while keeping the ultrasound measurement cycle as short as possible. However, there is a problem that power consumption increases. In contrast, uniform data need not be necessary in the entire period of a urine volume measurement period. Hereinafter, as an example of such a case, the following example will be described. That is, when urine volume in the bladder approaches the certain second threshold (alarm urine volume) prompting the measurement subject to urinate, that is, when the measured urine volume reaches the certain first threshold (pre-alarm urine volume) less than the alarm urine volume, in the case of shortening the urine volume measurement cycle in order to obtain more accurate urine volume in a period until the urine volume reaches the alarm urine volume, conversely, until urine volume in the bladder reaches the pre-alarm urine volume in the measurement modes, the measurement cycle is kept long to reduce power consumption.

First, the certain first threshold, which serves as a reference for changing the measurement cycle, will be described. As has been described above, the first threshold is a value for determining whether urine volume in the bladder is immediately before reaching the alarm urine volume. In the embodiment, the pre-alarm urine volume is input using the input unit 22 or calculated on the basis of the alarm urine volume. The pre-alarm urine volume set in this manner is stored in the memory 23.

The second comparator 24d compares the urine volume in the bladder, calculated by the retained urine volume calculator 24a, with the pre-alarm urine volume. In the case where the urine volume in the bladder is less than the pre-alarm urine volume, it is determined that the urine volume in the bladder is not immediately before reaching the alarm urine volume, and the urine volume in the bladder is measured at the certain first time interval without shortening the measurement cycle. In the embodiment, the first time interval here is one minute, as illustrated in Fig. 5. In contrast, in the case where the urine volume in the bladder is greater than or equal to the pre-alarm urine volume, it is determined that the urine volume in the bladder is immediately before reaching the alarm urine volume. The measurement cycle is shortened, and the urine volume in the bladder is measured at the certain second time interval. In the embodiment, the second time interval here is ten seconds.

As has been described above, the positioning mode is generally conducted before one of the measurement modes starts. In the embodiment, a measurement cycle (third cycle) in the case where urine volume in the bladder is less than the pre-alarm urine volume is longer than the measurement cycle (first cycle) in the positioning mode and the measurement cycle (second cycle) in the case where urine volume in the bladder is greater than or equal to the pre-alarm urine volume. Accordingly, positioning becomes accurate, and the measurement interval near the alarm urine volume becomes short, thereby implementing highly accurate measurement. Note that the measurement cycle (first cycle) at the time of positioning may be the same as the measurement cycle (second cycle) in the case where urine volume in the bladder is greater than or equal to the pre-alarm urine volume.

In addition, the number of ultrasound reflected waves used in one measurement in the case where urine volume in the bladder is less than the pre-alarm urine volume (third number) may be made less than that (second number) in the case where urine volume in the bladder is greater than or equal to the pre-alarm urine volume, but it is desirable that these numbers be the same number in order to ensure data precision. In the positioning mode, the number (first number) of ultrasound reflected waves used in one measurement is desirably less than the number (second number) of ultrasound reflected waves used in the case where the urine volume is greater than or equal to the pre-alarm urine volume.

The ultrasound controller 21 controls ultrasound transmitted from each ultrasound device 11 in order to be able to measure urine volume in the bladder every measurement cycle. That is, when urine volume in the bladder is less than the pre-alarm urine volume, each ultrasound device 11 transmits ultrasound at the certain first time interval. When urine volume in the bladder is greater than or equal to the pre-alarm urine volume, each ultrasound device 11 transmits ultrasound at the certain second time interval, which is shorter than the first time interval, at least until the urine volume reaches the alarm urine volume.

The display unit 27 displays the pre-alarm urine volume, stored in the memory 23, on the bar graph. At this time, the measurement cycle is different between the case in which urine volume in the bladder is less than the pre-alarm urine volume and the case in which urine volume in the bladder is greater than or equal to the pre-alarm urine volume. The bar graph display interval may be an interval in accordance with the first time interval, may be the second time interval, or a time interval different from these time intervals. In the case where the display interval is the first time interval, in a time slot where the measurement cycle is the first time interval, it is possible to display all measured urine volumes in the bladder as a bar graph. However, in a time slot where the measurement cycle is the second time interval, all urine volumes in the bladder greater than or equal to the pre-alarm urine volume cannot be displayed as a bar graph. At this time, data for each display interval is extracted from the urine volumes in the bladder, which are stored in the memory 23, and displayed on a bar graph. As a method of extracting this data, there is a method that extracts the maximum value among urine volumes in the bladder that are calculated within the display interval, or a method that calculates an average.

In contrast, in the case where the display interval is the second time interval, in a time slot where the measurement cycle is the second time interval, all urine volumes in the bladder that are greater than or equal to the pre-alarm urine volume are displayed as a bar graph. In a time slot where the measurement cycle is the first time interval, the resultant bar graph becomes one in which a measurement value at a time at which no measurement has been performed becomes missing. At this time, if the second time interval is an integer multiple of the first time interval, more actual measurement values can be displayed on a graph.

Next, on the basis of Fig. 13, a method of measuring urine volume using the ultrasonic urine volume measuring instrument 1 in the low power consumption mode will be described. First, the alarm urine volume is input by the measurement subject or the like to the input unit 22 (step S30). Next, the pre-alarm urine volume is set (step S31). At this time, the pre-alarm urine volume may be one input using the input unit 22 or one calculated by multiplying the alarm urine volume by a certain constant.

Next, each ultrasound device 11 transmits ultrasound toward the bladder of the measurement subject and receives its reflected waves, the ultrasound controller 21 converts the received reflected waves to reflected wave data, and the retained urine volume calculator 24a calculates urine volume in the bladder on the basis of the reflected wave data, thereby measuring urine volume in the bladder (step S32). The cycle of measuring the urine volume in the bladder at this time is the first time interval (one-minute interval). The measured urine volume in the bladder is displayed on the display unit 27, output to the output unit 25, or stored in the memory 23.

Next, the second comparator 24d compares the calculated urine volume in the bladder with the pre-alarm urine volume (step S33). In the case where the measured urine volume in the bladder is less than the pre-alarm urine volume, the ultrasonic urine volume measuring instrument 1 returns to step S32 and performs control to continuously transmit ultrasound to the bladder at the first time interval. Therefore, each ultrasound device 11 transmits ultrasound to the bladder at the first time interval until the urine volume in the bladder becomes greater than or equal to the pre-alarm urine volume, and urine volume in the bladder is measured.

In the case where the measured urine volume in the bladder is greater than or equal to the pre-alarm urine volume in step S33, since the urine volume in the bladder is immediately before reaching the alarm urine volume, the ultrasound controller 21 changes the measurement cycle to the second time interval (a measurement cycle of 10 seconds), and accordingly each ultrasound device 11 transmits ultrasound to the bladder at the second time interval and receives its reflected waves from the bladder. Conversion to reflected wave data by the ultrasound controller 21 and calculation of urine volume in the bladder by the retained urine volume calculator 24a are performed as in step S32 (step S34).

Next, the second comparator 24d compares the measured urine volume in the bladder with the alarm urine volume (step S35). In the case where the urine volume in the bladder is less than the alarm urine volume, the ultrasonic urine volume measuring instrument 1 returns to step S34 and performs control to continuously transmit ultrasound to the bladder at the second time interval and to measure urine volume in the bladder. In the case where the urine volume in the bladder is greater than or equal to the alarm urine volume, the alarm unit 26 produces an alarm (step S36).

As has been described above, the measurement cycle is changed in accordance with the calculated urine volume in the bladder in the low power consumption mode. Specifically, when it is determined that the urine volume in the bladder is immediately before reaching the alarm urine volume since the urine volume has become greater than or equal to the first threshold, the urine volume in the bladder can be more accurately calculated by shortening the measurement cycle. In contrast, when it is determined that the urine volume in the bladder is not immediately before reaching the alarm urine volume since the urine volume is less than the first threshold, the power consumption of the ultrasonic urine volume measuring instrument 1 can be reduced by elongating the measurement cycle.

In addition to shortening the measurement cycle as described above, the power consumption can be further reduced by causing a CPU included in the arithmetic unit 24 to enter a sleep state.

Fig. 14 is a block diagram of the ultrasonic urine volume measuring instrument 1 of the embodiment including a battery 100 and a timer unit 28 for causing the CPU to enter a sleep state or causing the CPU to recover. The timer unit 28 includes a power supply control circuit 101 and a timer clock circuit 102. In the case where the calculated urine volume in the bladder is greater than or equal to the pre-alarm urine volume, power from the battery 100 is supplied via the power supply control circuit 101 to the CPU included in the arithmetic unit 24, thereby causing the CPU to operate normally. On the basis of a count of the timer clock circuit 102, ultrasound transmission and calculation of urine volume in the bladder are performed at a short measurement cycle.

In contrast, in the case where the calculated urine volume in the bladder is less than the pre-alarm urine volume, the CPU enters a sleep state. At this time, power that is enough for keeping the CPU to be in a sleep state is supplied from the battery 100 to the CPU via the power supply control circuit 101. In response to an instruction to transmit ultrasound and to measure urine volume in the bladder the next time on the basis of a count of the timer clock circuit 102, power supplied from the power supply control circuit 101 to the CPU becomes great power for measurement, and the CPU recovers and operates normally.

In response to a certain input from the input unit 22 while the CPU is in a sleep state, it is desirable to cause the CPU to recover from a sleep state. It is thus desirable to supply a certain amount of power from the power supply control circuit 101 to the input unit 22 even when the CPU is in a sleep state.

The urine volume measurement interval is increased until urine volume in the bladder reaches the pre-alarm urine volume in the low power consumption measurement mode, and furthermore the CPU is caused to enter a sleep state, thereby reducing the power consumption.

(F) Recording of urination diary

Hereinafter, a urine volume management data generating and recording system using the ultrasonic urine volume measuring instrument will be described. This system is suitably used in designing a plan for training the bladder of a measurement subject (patient) with a urination disorder, such as overactive bladder. This system automatically generates and records urine volume management data such as a urination diary in which various types of data such as the measurement subject's retained urine volume (= urine volume in the bladder) at each measurement time, urination volume, and urination time are chronologically recorded. The urine volume management data may be generated using urine volume in the bladder calculated in (B) residual urine mode, (C) regular mode, (D) alarm mode, and (E) low power consumption mode described above.

Fig. 15 is a diagram that specifically illustrates the block diagram of Fig. 1 in order to describe a urine volume management data generating method. As illustrated in Fig. 15, a urine volume measurement system 1 in the embodiment is configured by a measuring instrument main body that mainly includes the ultrasound probe 10 and the controller 20. The controller 20 roughly includes the ultrasound controller 21, the input unit 22, the memory 23, the arithmetic unit 24, the output unit 25, and the display unit 27, and the schematic configurations thereof are as described in <Embodiment of Basic Configuration>. Thus, overlapping descriptions are omitted except when necessary.

As illustrated in Fig. 16, the input unit 22 includes a plurality of press-type operators (buttons), and pressing each of the buttons appropriately inputs input data.

The arithmetic unit 24 includes a third comparator 24e that compares urine volume in the bladder with a certain threshold, and the retained urine volume calculator 24a, which calculates urine volume in the bladder on the basis of the reflected wave data. The urine volume in the bladder calculated by the retained urine volume calculator 24a, and its measurement time are stored in a measurement data memory 23b in the memory 23. Note that the arithmetic unit 24 is also capable of calculating urine volume in the bladder at a specific time, such as urine volume in the bladder corresponding to a later-described micturition time (urine volume in the bladder upon micturition), on the basis of urine volume in the bladder and its measurement time stored in the measurement data memory 23b.

The display unit 27 displays, on a screen, various types of data such as urine volume in the bladder at each time. As illustrated by way of example in Fig. 16, for example, the display unit 27 displays a bar graph in which time is plotted on the horizontal axis, and urine volume in the bladder is plotted on the vertical axis. It can be detected from the bar graph the urine volume in the bladder and its temporal changes. On the screen, various types of data including a later-described MAX value are displayed.

Regarding the method of calculating urine volume retained in the bladder, the calculation method using equations (1) and (2) discussed in <Embodiment of Basic Configuration> is used to calculate urine volume EU in the bladder every measurement cycle. The calculated urine volume EU is chronologically stored in the memory 23.

The urine volume EU in the bladder calculated in this manner is chronologically stored in the measurement data memory 23b. Fig. 17 represents, in the form of a table, urine volume in the bladder at each time (measurement cycle that is every minute) stored in the measurement data memory 23b.

Next, using Fig. 15 and Figs. 16 to 24, the urine volume measurement system 1 of the embodiment will be more specifically described. Note that generation and recording of urine volume management data in the case of measurement in the above-described regular measurement mode will be described here.

The input unit 22 includes a micturition input unit used by the measurement subject to input a micturition signal at the time the measurement subject feels micturition. As illustrated in Fig. 16, the micturition input unit includes any one of the multiple buttons 43 included in the input unit 22. It is configured that pressing the button inputs a micturition signal to the micturition input unit, and a micturition time corresponding to the input time of the micturition signal (pressing time) is recorded in the measurement data memory 23b. Note that Fig. 18 represents, in the form of a table, a micturition time recorded in the measurement data memory 23b.

At the time a micturition signal is input to the micturition input unit, as has been described above, its micturition time is stored in the measurement data memory 23b, and urine volume in the bladder upon micturition, which corresponds to the micturition time, is calculated by the arithmetic unit 24. The urine volume in the bladder upon micturition is compared by the third comparator 24e in the arithmetic unit 24 with a certain alarm threshold (alarm urine volume), and, as illustrated by way of example in Fig. 19A and Fig. 19B, the urine volume in the bladder upon micturition is displayed on the display unit 27. Although the alarm urine volume compared by the third comparator 24e may be, for example, a predetermined value, the alarm urine volume may be one input by the measurement subject or a health professional via the input unit 22, which is an appropriate value.

In the embodiment, the urine volume in the bladder upon micturition is calculated on the basis of urine volume in the bladder in a measurement cycle immediately prior to the time of inputting a micturition signal to the micturition input unit. Specifically, the urine volume in the bladder upon micturition is the most recent urine volume in the bladder stored in the measurement data memory 23b at the time of inputting a micturition signal to the micturition input unit. When a measurement error has been caused by a postural change or the like and there is no urine volume in the bladder calculated within a certain time (such as one minute) prior to the input of a micturition signal, no urine volume in the bladder upon micturition is recorded.

The third comparator 24e compares the value of urine volume in the bladder upon micturition with the alarm urine volume, and, in the case where the urine volume in the bladder upon micturition does not exceed a certain ratio (such as 80%) of the alarm urine volume, the urine volume in the bladder upon micturition is displayed using, for example, a numeral, thereby enabling the measurement subject to perceive that there is no need to urinate. In this manner, inputting a micturition signal to the micturition input unit (pressing a button) causes the urine volume in the bladder upon micturition to be displayed as a numeral via the display unit 27, and the result of comparison performed by the third comparator 24e is displayed as a numeral. For example, even in the case where the measurement subject feels micturition although urine in the bladder is only of a little volume, the measurement subject can be notified that there is no need to urinate. Accordingly, the time for filling the bladder with urine can be gradually increased, thereby expanding a urination interval.

Regarding the urine volume in the bladder upon micturition, the urine volume in the bladder upon micturition may be obtained on the basis of a plurality of values of urine volume in the bladder in a measurement cycle immediately prior to the time of inputting a micturition signal. In this case, for example, at the time of inputting a micturition signal to the micturition input unit, the urine volume in the bladder upon micturition may be obtained as an average urine volume in the bladder from a plurality of values of urine volume in the bladder, excluding the maximum value and the minimum value, stored in the measurement data memory 23b. In addition, at the time of inputting a micturition signal, the urine volume in the bladder upon micturition may be obtained by performing interpolation on a plurality of values of urine volume in the bladder stored in the measurement data memory 23b.

Alternatively, the urine volume in the bladder upon micturition may be the value of urine volume in the bladder calculated by the retained urine volume calculator 24a on the basis of reflected waves of ultrasound transmitted by each ultrasound device 11 toward the bladder at the time of inputting a micturition signal to the micturition input unit. In this case, the urine volume in the bladder upon micturition is not based on the past urine volumes in the bladder estimated and stored in units of measurement cycles, but is the value of urine volume in the bladder measured at a time point at which the button corresponding to the micturition input unit is pressed.

The value of urine volume in the bladder of the measurement subject is monitored, and, among urine volumes in the bladder calculated by the retained urine volume calculator 24a, the maximum urine volume in the bladder is recorded as a MAX value in the memory 23. Specifically, the arithmetic unit 24 compares urine volume in the bladder obtained every measurement cycle with a MAX value stored in the memory 23, and, in the case where the urine volume in the bladder exceeds the MAX value, that value of urine volume in the bladder is recorded as a new MAX value in the memory 23. In addition, the MAX value is read out from the memory 23, and is displayed, for example, as illustrated in Fig. 16, on the display unit 27 (upper right-hand corner of the screen). In addition, at the time of outputting from the output unit 25, the MAX value may be read out from the memory 23, and may be output along with, for example, the urine volume in the bladder and micturition time.

As a result of measurements conducted for a few hours to one day, the MAX value becomes the value of the rough maximum urine volume that can be retained in the bladder of the measurement subject, and serves as a criterion for training the bladder afterwards. In accordance with displaying of the MAX value on the display unit 27 or the like, the measurement subject becomes aware of the fact that more urine can be held in his/her bladder.

In addition, the arithmetic unit 24 of the embodiment includes a management data generator 24f that generates, on the basis of data stored in the memory 23, urine volume management data by chronologically arranging at least retained urine data for each certain measurement cycle, micturition data including a micturition time, and urination data including urination volume calculated on the basis of the retained urine data, and its urination time. The management data generator 24f generates urine volume management data by combining, for example, urine volume in the bladder at each time and a micturition time stored in the measurement data memory 23b. It is configured that the urine volume management data is output from the output unit 25 or displayed on the display unit 27.

The urine volume management data can be used as a urination diary for a measurement subject with overactive bladder or the like. For example, an example of urine volume management data includes, as illustrated in Fig. 20, urine volume in the bladder at each time (measurement cycle that is every minute), the time at which the measurement subject feels micturition and inputs a micturition signal to the micturition input unit, and urination volume and its urination time that are chronologically represented in the form of a table. In the embodiment, a MAX value serving as the maximum urine volume in the bladder is represented in the urine volume management data.

The arithmetic unit 24 calculates the urination volume and its urination time on the basis of a time at which the urine volume in the bladder greatly decreases, from temporal changes of the urine volume in the bladder at each time, stored in the measurement data memory 23b. Specifically, a urination volume calculator 24g in the arithmetic unit 24 compares urine volume in the bladder at a certain time, stored in the measurement data memory 23b, with urine volume in the bladder at the next time to obtain a urine volume difference, searches for a time at which that difference becomes greater than or equal to a certain urination threshold stored in the memory 23, calculates the urine volume difference greater than or equal to the urination threshold as urination volume, and displays the urination volume on the display unit 27 or outputs the urination volume to the output unit 25. The urination threshold is set in order to avoid determination of a small urination volume decrease based on a measurement error as "urination". Note that the urination threshold may be set in advance, or the measurement subject or the like may set the urination threshold through the input unit 22. An example of the urination threshold includes 50 cc, though the urination threshold is not limited to this value.

In addition, the urination time may be recorded on the basis of an input from the measurement subject. That is, the time at which the measurement subject operates a button (one of the buttons 43) allocated to record the urination time is recorded as the urination time. The urination volume at this time can be obtained from the amount of reduction in the urine volume in the bladder within a certain time before and after the button operation. The inputting and recording of the urination time may be performed in response to an input of the urination start time and an input of the urination end time, respectively. In this case, the urination volume can also be obtained by calculating, by the arithmetic unit 24, a difference between the urine volume in the bladder at the urination start or closest to the urination start and the urine volume in the bladder at the urination end or closest to the urination end.

In addition, the function of determining whether urination that causes a reduction in the urine volume in the bladder is voluntary urination or leakage of urine may be provided. For example, in the case where urination recording is performed in response to a button operation performed by the measurement subject, if there is a reduction in urine volume in the bladder in a state where there is no urination recording in response to a button operation, it can be recorded that the reduction is leakage of urine. Specifically, the arithmetic unit 24 searches for a time at which the urine volume in the bladder greatly decreases, from temporal changes of the urine volume in the bladder at each time, stored in the measurement data memory 23b, calculates a difference between that time and a prior urination recorded time, and, in the case where the difference exceeds a certain time, it can be determined that the decrease is leakage of urine. Alternatively, the arithmetic unit 24 may calculate a difference between a time at which there is a reduction in the urine volume in the bladder and a prior micturition recorded time, and, in the case where the difference exceeds a certain time, it can be similarly determined that the reduction is leakage of urine. At this time, referring to Figs. 20, 21, 23, and 24, whether the urination volume is leakage or not can be output/displayed.

Although the micturition time in Fig. 20 is the time at which a micturition signal is input to the micturition input unit, the micturition time may be displayed as a time that is near the time of inputting a micturition signal and that is when the urine volume in the bladder is calculated by the retained urine volume calculator 24a (see Fig. 21). Alternatively, as illustrated in Fig. 22, a bar graph may be represented in which urine volume in the bladder is plotted on the vertical axis and time is plotted on the horizontal axis, and the micturition time may be indicated using a symbol such as an arrow. In Fig. 22, the value of urine volume in the bladder obtained every five minutes is displayed as a bar graph, and the value of urine volume in the bladder suddenly decreases between 11:20 and 11:30. From this point, it can be visually recognized that urination has taken place between these points of time.

In this example, the urine volume management data is generated and recorded on the basis of measurement results in the regular measurement mode. For example, the measurement cycle is shortened from the time of inputting a micturition signal to the micturition input unit to the urination time (such as from 11:17:00 to 11:24:00 in Fig. 21), thereby accurately detecting urine volume retained in the bladder after micturition has been felt. In other certain periods, the urine volume measurement cycle is elongated, thereby reducing power consumption.

In the embodiment, urine volume management data can be displayed in the form of a table illustrated in Fig. 23 by comparing the urine volume in the bladder at each time and the micturition time stored in the measurement data memory 23b to extract urine volume in the bladder corresponding to the micturition time, calculating the urination time using the above-described urination volume calculating method, and extracting urine volume in the bladder at the urination time. Although the urine volume in the bladder at the urination time is extracted in Fig. 23, in addition to that, the urine volume in the bladder calculated immediately prior to the urination time may also be extracted and output as in Fig. 24. According to such urine volume management data, only the micturition time, the urination volume, and the urine volume in the bladder at the urination time are displayed, and, since only these activities serving as points in a urination diary are represented, these items of data can be effectively used when the patient visits a clinic, for example.

The urine volume management data generated by the management data generator 24f can also be stored in, for example, a management data memory 23c in the memory 23, and the urine volume management data can be read out from the management data memory 23c as needed.

Although only the presence of micturition is recorded here, the micturition strength (urgency) may be recorded in five steps ranging from 1 to 5. For example, when the measurement subject is asked to press the micturition input unit five times when the micturition strength is 5 (very strong) and to press the micturition input unit once when the micturition strength is 1 (there is micturition), the micturition strength can be determined by the ultrasonic urine volume measuring instrument. In this case, a numeral or a symbol indicating the micturition strength can be output/displayed in the micturition column in Figs. 23 and 24. In the above-described embodiment according to the present invention, all elements of the ultrasound probe 10 and the controller 20 need not be integrally formed. For example, the output unit 25, the alarm unit 26, the display unit 27, and the input unit 22 in the controller 20 may be configured in a separate information terminal. In this case, the memory 23 is also provided in the information terminal, and it is only necessary to configure that measurement data is transmitted/received using WiFi or Bluetooth with a portion that performs ultrasound measurement.

In the ultrasonic urine volume measurement system 1, when there is an occasion to generate urine volume management data such as a urination diary, it is not necessary for the measurement subject to measure the urine volume in the bladder (urination volume) and the micturition time every time, and the urine volume management data is automatically generated. Thus, the system 1 exerts a smaller load on the measurement subject and can generate urine volume management data easily and accurately. Since urine volume management data generated in the embodiment includes the urine volume in the bladder at each time, the micturition time, and the urination volume and its time that are chronologically arranged, the data is generated in a form that enables the measurement subject to visually understand the relationship between micturition and the urine volume in the bladder (urination volume) at each time. The urine volume management data also enables a health professional to understand the measurement subject's subjective symptom and the actual bladder function, and to design an appropriate bladder training plan suitable for the measurement subject.

Next, on the basis of Fig. 25, an embodiment of generating urine volume management data using an external communication terminal will be described. Hereinafter, along with the configuration thereof, a urine volume management data generating method according to the present invention will be described. In order to prevent overlapping descriptions, the same reference numerals are given to the same components as above and to effects based on these components to omit detailed descriptions.

A urine volume measurement system 30 using the external communication terminal is different from the first embodiment in the point that the urine volume measurement system 30 includes a measuring instrument main body 31 (urine volume measurement device) including each ultrasound device 11, and an external communication terminal 32 (urine volume management data generating terminal) connected to the measuring instrument main body 31.

The external communication terminal 32 is not limited to a particular device and may be, for example, a smart phone, a mobile phone, a tablet terminal, or a personal computer.

As illustrated in Fig. 25, the measuring instrument main body 31 at least includes the main-body-side arithmetic unit 24 out of the above-mentioned arithmetic unit, and a first communication unit 33 that transmits the urine volume in the bladder calculated by the main-body arithmetic unit 24 and its measurement time to the external communication terminal 32. The measuring instrument main body 31 also includes the ultrasound controller 21, and a first memory 34 that stores the urine volume in the bladder and the measurement time. In the embodiment, it is assumed that the main-body-side arithmetic unit 24 includes the retained urine volume calculator 24a which calculates the urine volume in the bladder on the basis of the reflected wave data.

In contrast, the external communication terminal 32 includes a terminal-side arithmetic unit 37 out of the above-mentioned arithmetic unit, a second communication unit 35 (receiving unit) that performs transmission and reception with the first communication unit 33, the micturition input unit, a management data generator 37b, and a second memory 36 including a measurement data memory 36a that stores the urine volume in the bladder and its measurement time received via the second communication unit 35, and the micturition time. Furthermore, in the case of receiving an input of the urination time from the measurement subject, urination time data (may be the urination start time or the urination end time) can be stored in a memory (not illustrated). The external communication terminal 32 includes the input unit 22, the display unit 27, and the output unit 25. In the embodiment, it is assumed that the terminal-side arithmetic unit 37 includes a third comparator 37a that compares the urine volume in the bladder upon micturition with the alarm urine volume, the management data generator 37b which generates urine volume management data, and a urination volume calculator 37c that calculates urination volume and its urination time.

In response to an instruction from the external communication terminal 32 (such as an input operation of the input unit 22), data such as the urine volume in the bladder stored in the first memory 34 of the measuring instrument main body 31 is transmitted to the external communication terminal 32 from the first communication unit 33 via the second communication unit 35. Communication between the measuring instrument main body 31 and the external communication terminal 32 is performed using a wireless communication system such as Wi-Fi (Wireless Fidelity), Bluetooth, or NFC (Near Field Communication). Regarding the data transmission/reception timing between the measuring instrument main body 31 and the external communication terminal 32, the timing may be constant or every certain time. Alternatively, the timing may be the time of inputting to the input unit 22 of the external communication terminal 32. It is assumed that transmission/reception is performed at an appropriate timing.

As illustrated in Fig. 25, the external communication terminal 32 receives data regarding the urine volume in the bladder and its measurement time, transmitted from the measuring instrument main body 31, via the second communication unit 35, and stores the data in the measurement data memory 36a in the second memory 36. In response to an input of a micturition signal to the micturition input unit, the micturition time is stored in the measurement data memory 36a, and a urine volume value upon micturition corresponding to the micturition time is read out from the measurement data memory 36a.

In the external communication terminal 32, the management data generator 37b in the terminal-side arithmetic unit 37 is configured to generate urine volume management data such as a urination diary on the basis of data stored in the second memory 36. For example, the data is displayed on the display unit 27 in a form such as that discussed above with reference to Figs. 20 to 22, or may be output from the output unit 25. For example, as in Fig. 26, on the basis of the above-mentioned data, the main-body-side arithmetic unit 24 can calculate and display the MAX value per specified three days, and the number of urinations and the number of micturitions per specified day. Furthermore, as has been described above, the urination time may be included in the urine volume management data. In addition, the time at which the urine volume in the bladder decreases may be detected by the main-bod-side arithmetic unit 24 and, based on that, urination volume may be stored. In addition, the time difference between the time at which the urine volume decreases and the urination time (urination start time or urination end time) input by the measurement subject or the micturition time may be calculated, and that time difference may be included in the urine volume management data. Furthermore, whether a reduction in the urine volume in the bladder is done consciously or not, that is, whether the reduction is leakage of urine, may be determined on the basis of the time difference, and data indicating the determination result may be included in the urine volume management data.

Calculation and display data of the external communication terminal 32 are configured to be arbitrarily selectable by a user such as a health professional.

By providing a cloud management data memory 38, the generated urine volume management data can be saved or read out using the second communication unit 35 via Wi-Fi or the like. Alternatively, a management data memory may be provided in a storage area in a memory of the measuring instrument main body 31 or the external communication terminal 32.

In the example illustrated in Fig. 25, the input unit 22, the micturition input unit, the display unit 27, the output unit 25, the third comparator 37a, the management data generator 37b, and the urination volume calculator 37c are provided on the external communication terminal 32 side. However, the configuration is not limited to this, and these components may be provided on the measuring instrument main body 31 side.

The ultrasonic urine volume measuring instrument 1 of the embodiment can also be used as a device for toilet training or bladder training. Toilet training is performed to train a child who has passed the age of one year old to acquire the habit of using the toilet. In general, toilet training is done by predicting the urination timing and guiding the child to the toilet at that timing. However, urine volume in the bladder varies depending on food and drink or temperature, and it is thus difficult to predict the urination timing. As a result, there are cases in which the child is not guided to the toilet at an appropriate timing. Since the ultrasonic urine volume measuring instrument 1 monitors urine volume in the bladder of a measurement subject, it becomes possible to guide the measurement subject to the toilet at an appropriate timing. Particularly, since a timing at which the bladder is fully filled with urine is reported as an alarm on the basis of the actually measured urine volume in the bladder in the alarm mode or the low power consumption mode, the measurement subject can be accurately notified of a urination timing, and toilet training can thus be effectively performed.

Bladder training is performed to train a patient with overactive bladder or the like, who experiences frequent urination, or an enuretic child to hold back urination as long as possible, thereby increasing the volume of urine that can be retained in the bladder. In this training, it is important to know the difference in the sense that, when one feels micturition, whether that micturition is really a cue felt by having the bladder filled with urine or is simply micturition caused by a tense bladder. According to the ultrasonic urine volume measuring instrument 1 of the embodiment, since urine volume in the bladder is actually measured, the measurement subject can understand whether it is a timing to hold urination or not. In particular, displaying urine volume at a point of time at which the measurement subject feels micturition and the maximum urine volume in the bladder enables the measurement subject to more accurately determine whether he/she should hold urination, and hence bladder training can be efficiently performed. Specifically, the training is performed by the following steps.

<Step 300> When the measurement subject feels micturition, the measurement subject inputs a micturition signal to the ultrasonic urine volume measuring instrument 1, as described above.

<Step 301> The ultrasonic urine volume measuring instrument 1 displays the urine volume in the bladder upon micturition and the maximum urine volume in the bladder.

<Step 302> On the basis of the displayed urine volume in the bladder upon micturition and the maximum urine volume in the bladder, the measurement subject determines whether to hold urination.

The embodiment has the following characteristics.

(1) An ultrasonic urine volume measuring instrument comprising:

an ultrasound probe that is placed on stomach of a measurement subject, and that includes an ultrasound device that transmits ultrasound toward bladder of the measurement subject at a certain measurement cycle and receives a wave reflected from a wall of the bladder;
an ultrasound controller transmission and reception ultrasound and its reflected wave, the transmission and reception being performed by the ultrasound device;
an arithmetic unit that calculates, on the basis of the reflected wave, urine volume in the bladder said every measurement cycle; and
a display unit that chronologically displays, as a graph, the urine volume calculated by the arithmetic unit,
the ultrasonic urine volume measuring instrument further comprising:

a mode selector capable of selecting one of a measurement mode of measuring urine volume while the ultrasound probe is placed at a certain measurement position on the stomach of the measurement subject, and a positioning mode of measuring urine volume while moving the ultrasound probe to a plurality of tentative measurement positions on the stomach in order to determine the measurement position suitable for placing the ultrasound probe; and
an index value memory that stores a maximum value among urine volumes at the plurality of tentative measurement positions, calculated by the arithmetic unit, while the positioning mode is being selected, and/or an allowance value determined on the basis of the maximum value,
wherein the maximum value and/or the allowance value is displayed as a positioning index value for positioning the ultrasound probe, on the graph on the display unit.

(2) An ultrasonic urine volume measuring instrument comprising:

an ultrasound probe that is placed at a measurement position on stomach of a measurement subject, and that includes an ultrasound device that transmits ultrasound toward bladder of the measurement subject and receives a wave reflected from a wall of the bladder;
an ultrasound controller that controls transmission and reception of ultrasound and its reflected wave, the transmission and reception being performed by the ultrasound device; and
an arithmetic unit that calculates, on the basis of the reflected wave, urine volume in the bladder,
wherein the arithmetic unit calculates, in a positioning mode of determining the measurement position suitable for placing the ultrasound probe, urine volume in the bladder while the ultrasound probe is arranged at a plurality of tentative measurement positions on the stomach, and
wherein the ultrasonic urine volume measuring instrument further comprises:

an index value memory that stores a maximum value among urine volumes calculated by the arithmetic unit during the positioning mode and/or an allowance value determined on the basis of the maximum value, and
a comparator that detects, in the positioning mode, a difference between the urine volume calculated by the arithmetic unit and the maximum value and/or the allowance value stored in the index value memory.

(3) The ultrasonic urine volume measuring instrument of (2) further comprising a reporting unit that produces an alarm on the basis of a result of comparison performed by the comparator.
(4) The ultrasonic urine volume measuring instrument of (2) or (3) further comprising a display unit that displays, as a graph, urine volume values calculated at a plurality of times by the arithmetic unit,
wherein the display unit can change a time scale of the graph display between the positioning mode and a measurement mode of measuring urine volume in the bladder while the ultrasound probe is placed at the measurement position.
(5) An ultrasonic urine volume measuring instrument comprising:

an ultrasound probe that is placed at a measurement position on stomach of a measurement subject, and that includes an ultrasound device that transmits ultrasound toward bladder of the measurement subject and receives a wave reflected from a wall of the bladder;
an ultrasound controller that controls transmission and reception of ultrasound and its reflected wave, the transmission and reception being performed by the ultrasound device; and
an arithmetic unit that calculates, on the basis of the reflected wave, urine volume in the bladder,
wherein the arithmetic unit calculates, in a positioning mode of determining the measurement position suitable for placing the ultrasound probe, urine volume by using a calculation method different from that in a measurement mode of calculating urine volume at the measurement position, and
wherein the urine volume calculation method in the positioning mode is more reactive to a change in the position of the ultrasound probe than the urine volume calculation method in the measurement mode.

(6) In the ultrasonic urine volume measuring instrument of (5), the arithmetic unit calculates urine volume on the basis of a plurality of reflected waves that are consecutively received, and the number of reflected waves used in calculating urine volume is less in the positioning mode than in the measurement mode.
(7) An ultrasonic urine volume measuring instrument comprising:

an ultrasound probe that includes an ultrasound device that transmits ultrasound toward bladder of a measurement subject at a certain measurement cycle and receives a wave reflected from a wall of the bladder;
an ultrasound controller that controls transmission and reception of ultrasound and its reflected wave, the transmission and reception being performed by the ultrasound device; and
an arithmetic unit that calculates, on the basis of the reflected wave, urine volume in the bladder said every measurement cycle,
wherein the arithmetic unit includes a comparator that compares the calculated urine volume in the bladder with a certain first threshold,
wherein the measurement cycle of ultrasound transmitted from the ultrasound device is longer in a case where the urine volume in the bladder compared by the comparator is less than the first threshold than that in a case where the urine volume is greater than or equal to the first threshold, and
wherein the ultrasonic urine volume measuring instrument further comprises a timer unit capable of causing, in a case where the urine volume in the bladder compared by the comparator is less than the first threshold, the arithmetic unit to enter a sleep state during each measurement cycle in order to reduce power consumption, and causing the arithmetic unit to recover every measurement cycle.

(8) An ultrasonic urine volume measuring instrument comprising:

an ultrasound probe that includes an ultrasound device that transmits ultrasound toward bladder of a measurement subject at a certain measurement cycle and receives a wave reflected from a wall of the bladder;
an ultrasound controller that controls transmission and reception of ultrasound and its reflected wave, the transmission and reception being performed by the ultrasound device; and
an arithmetic unit that calculates, on the basis of the reflected wave, urine volume in the bladder said every measurement cycle,
the ultrasonic urine volume measuring instrument further comprising a mode selector capable of selecting one of a measurement mode of measuring urine volume while the ultrasound probe is placed at a certain measurement position on stomach of the measurement subject, and a positioning mode of measuring urine volume while moving the ultrasound probe to a plurality of tentative measurement positions on the stomach in order to determine the measurement position suitable for placing the ultrasound probe in the measurement mode,
wherein the arithmetic unit includes a comparator that compares the calculated urine volume in the bladder with a certain first threshold,
wherein the ultrasound controller is capable of:

causing, in the positioning mode, the ultrasound device to transmit ultrasound at a certain first cycle,
causing, in the measurement mode, the ultrasound device to transmit ultrasound at a certain second cycle in a case where the urine volume in the bladder compared by the comparator is greater than or equal to the first threshold, and
causing, in the measurement mode, the ultrasound device to transmit ultrasound at a third cycle longer than the first cycle and the second cycle in a case where the urine volume in the bladder compared by the comparator is less than the first threshold.

(9) An ultrasonic urine volume measuring instrument comprising:

an ultrasound probe that includes an ultrasound device that transmits ultrasound toward bladder of a measurement subject at a certain measurement cycle and receives a wave reflected from a wall of the bladder;
an ultrasound controller that controls transmission and reception of ultrasound and its reflected wave, the transmission and reception being performed by the ultrasound device; and
an arithmetic unit that calculates, on the basis of the reflected wave, urine volume in the bladder said every measurement cycle,
the ultrasonic urine volume measuring instrument further comprising a mode selector capable of selecting one of a measurement mode of measuring urine volume while the ultrasound probe is placed at a certain measurement position on stomach of the measurement subject, and a positioning mode of measuring urine volume while moving the ultrasound probe to a plurality of tentative measurement positions on the stomach in order to determine the measurement position suitable for placing the ultrasound probe in the measurement mode,
wherein the arithmetic unit includes a comparator that compares the calculated urine volume in the bladder with a certain first threshold, and
wherein the arithmetic unit is capable of calculating, in the positioning mode, urine volume in the bladder on the basis of a first number of reflected waves chronologically received at a certain first cycle,
measuring, in the measurement mode, in a case where the urine volume in the bladder compared by the comparator is greater than or equal to the first threshold, urine volume in the bladder on the basis of a certain second number of reflected waves chronologically received at a certain second cycle, the second number being greater than the first number, and
measuring, in the measurement mode, in a case where the urine volume in the bladder compared by the comparator is less than the first threshold, urine volume in the bladder on the basis of a plurality of reflected waves chronologically received at a certain third cycle that is longer than the first cycle and the second cycle.

(10) In the ultrasonic urine volume measuring instrument of any one of (7) to (9),

the comparator compares the calculated urine volume in the bladder with a certain second threshold that is greater than the first threshold, and
the ultrasonic urine volume measuring instrument further comprises a reporting unit that reports, in a case where the urine volume in the bladder compared by the comparator is greater than or equal to the second threshold, that the urine volume is greater than or equal to the second threshold.

(11) The ultrasonic urine volume measuring instrument further comprising:

a micturition input unit used by the measurement subject to input a micturition signal at a time the measurement subject feels micturition;
a measurement data memory that stores the urine volume in the bladder calculated by the arithmetic unit, its measurement time, and a micturition time corresponding to an input time of the micturition signal;
a maximum urine volume memory that stores maximum urine volume in the bladder that is a maximum value among urine volumes in the bladder calculated by the arithmetic unit in a certain period; and
a display unit that displays, at the time of inputting the micturition signal, retained urine volume upon micturition that is retained urine volume corresponding to the micturition time, and the maximum urine volume in the bladder.

(12) In the ultrasonic urine volume measuring instrument of (11), the retained urine volume upon micturition is urine volume in the bladder calculated by the arithmetic unit in a certain time prior to the time of inputting the micturition signal.
(13) The ultrasonic urine volume measuring instrument of (11) or (12) further comprising:

a urination start input unit used by the measurement subject to input a urination start signal before the measurement subject starts urination; and
a urination end input unit used by the measurement subject to input a urination end signal after the measurement subject ends urination,
wherein the measurement data memory stores a urination start time and a urination end time respectively corresponding to an input time of the urination start signal and an input time the urination end signal.

(14) A urine volume management data generating and displaying method comprising: a step of generating, on the basis of urine volume in the bladder and its measurement time, and the micturition time, stored in the ultrasonic urine volume measuring instrument according to any one of (11) to (13), urine volume management data by chronologically arranging at least urine-volume-in-bladder data including the urine volume in the bladder and its measurement time, and micturition data including the micturition time; and
a step of displaying at least part of the urine volume management data in the form of a table or a graph.
(15) In the method of (14), the urine volume management data further includes urination time data at least including one of a urination start time and a urination end time.
(16) The method of (15) further comprising:

a step of calculating the number of micturitions that is the number of micturition signals in a certain period on the basis of the micturition data, and the number of urinations that is the number of urination start signals or urination end signals in a certain period on the basis of the urination time data; and
a step of displaying the maximum urine volume in the bladder, the number of micturitions, and the number of urinations.

(17) The method of any one of (14) to (16) further comprising a step of searching the urine-volume-in-bladder data for a urine volume decrease time at which urine volume in the bladder decreases with time, and storing the amount of decrease as urination volume,
wherein the urine volume management data includes the urination volume.
(18) The method of (17) further comprising a step of calculating a time difference between each urine volume decrease time and the urination time or the micturition time,
wherein the urine volume management data includes the time difference or data calculated from the time difference.
(19) In any one of above-described (1) to (18), urine volume in the bladder displayed on the display unit is urine volume used in at least one of toilet training and bladder training.
(20) A bladder training method using an ultrasonic urine volume measuring instrument that is placed on stomach of a measurement subject, that transmits ultrasound toward bladder of the measurement subject, and that calculates urine volume in the bladder, the method comprising;

(a) a step of storing maximum urine volume in the bladder that is a maximum value among urine volume values measured in a certain period by the ultrasonic urine volume measuring instrument;
(b) a step of inputting, when the measurement subject feels micturition, a micturition signal to the ultrasonic urine volume measuring instrument;
(c) a step of displaying urine volume in the bladder at an input time of the micturition signal, and the maximum urine volume in the bladder on the ultrasonic urine volume measuring instrument; and
(d) determining whether it is necessary to urinate, on the basis of the displayed urine volume in the bladder and the displayed maximum urine volume in the bladder.

[Reference Signs List]

| | |
|---|---|
| 1 | ultrasonic urine volume measuring instrument |
| 10 | ultrasound probe |
| 11 | ultrasound devices |
| 21 | ultrasound controller |
| 22a | mode selector |
| 24 | arithmetic unit |
| 24d | second comparator |
| 23b, 36a | measurement data memory |

## Claims

**1.** An ultrasonic urine volume measuring instrument comprising:

an ultrasound probe that includes an ultrasound device that transmits ultrasound toward bladder of a measurement subject at a certain measurement cycle and receives a wave reflected from a wall of the bladder;
an ultrasound controller that controls transmission and reception of ultrasound and its reflected wave, the transmission and reception being performed by the ultrasound device; and
an arithmetic unit that calculates, on the basis of the reflected wave, urine volume in the bladder said every measurement cycle,
wherein the arithmetic unit includes a comparator that compares the calculated urine volume in the bladder with a certain first threshold,
wherein the measurement cycle of ultrasound transmitted from the ultrasound device is longer in a case where the urine volume in the bladder compared by the comparator is less than the first threshold than that in a case where the urine volume is greater than or equal to the first threshold, and
wherein the ultrasonic urine volume measuring instrument further comprises a timer unit capable of causing, in a case where the urine volume in the bladder compared by the comparator is less than the first threshold, the arithmetic unit to enter a sleep state during each measurement cycle in order to reduce power consumption, and causing the arithmetic unit to recover every measurement cycle.

**2.** An ultrasonic urine volume measuring instrument comprising:

an ultrasound probe that includes an ultrasound device that transmits ultrasound toward bladder of a measurement subject at a certain measurement cycle and receives a wave reflected from a wall of the bladder;
an ultrasound controller that controls transmission and reception of ultrasound and its reflected wave, the transmission and reception being performed by the ultrasound device; and
an arithmetic unit that calculates, on the basis of the reflected wave, urine volume in the bladder said every measurement cycle,
the ultrasonic urine volume measuring instrument further comprising a mode selector capable of selecting one of a measurement mode of measuring urine volume while the ultrasound probe is placed at a certain measurement position on stomach of the measurement subject, and a positioning mode of measuring urine volume while moving the ultrasound probe to a plurality of tentative measurement positions on the stomach in order to determine the measurement position suitable for placing the ultrasound probe in the measurement mode,
wherein the arithmetic unit includes a comparator that compares the calculated urine volume in the bladder with a certain first threshold,
wherein the ultrasound controller is capable of:

causing, in the positioning mode, the ultrasound device to transmit ultrasound at a certain first cycle,
causing, in the measurement mode, the ultrasound device to transmit ultrasound at a certain second cycle in a case where the urine volume in the bladder compared by the comparator is greater than or equal to the first threshold, and
causing, in the measurement mode, the ultrasound device to transmit ultrasound at a third cycle longer than the first cycle and the second cycle in a case where the urine volume in the bladder compared by the comparator is less than the first threshold.

**3.** An ultrasonic urine volume measuring instrument comprising:

an ultrasound probe that includes an ultrasound device that transmits ultrasound toward bladder of a measurement subject at a certain measurement cycle and receives a wave reflected from a wall of the bladder;
an ultrasound controller that controls transmission and reception of ultrasound and its reflected wave, the transmission and reception being performed by the ultrasound device; and
an arithmetic unit that calculates, on the basis of the reflected wave, urine volume in the bladder said every measurement cycle,
the ultrasonic urine volume measuring instrument further comprising a mode selector capable of selecting one of a measurement mode of measuring urine volume while the ultrasound probe is placed at a certain measurement position on stomach of the measurement subject, and a positioning mode of measuring urine volume while moving the ultrasound probe to a plurality of tentative measurement positions on the stomach in order to determine the measurement position suitable for placing the ultrasound probe in the measurement mode,
wherein the arithmetic unit includes a comparator that compares the calculated urine volume in the bladder with a certain first threshold, and
wherein the arithmetic unit is capable of calculating, in the positioning mode, urine volume in the bladder on the basis of a first number of reflected waves chronologically received at a certain first cycle,
measuring, in the measurement mode, in a case where the urine volume in the bladder compared by the comparator is greater than or equal to the first threshold, urine volume in the bladder on the basis of a certain second number of reflected waves chronologically received at a certain second cycle, the second number being greater than the first number, and
measuring, in the measurement mode, in a case where the urine volume in the bladder compared by the comparator is less than the first threshold, urine volume in the bladder on the basis of a plurality of reflected waves chronologically received at a certain third cycle that is longer than the first cycle and the second cycle.

**4.** The ultrasonic urine volume measuring instrument according to Claim 2 or 3, wherein the first cycle and the second cycle are the same cycle.

**5.** The ultrasonic urine volume measuring instrument according to any one of Claims 1 to 3,
wherein the comparator further compares the calculated urine volume in the bladder with a certain second threshold that is greater than the first threshold, and
wherein the ultrasonic urine volume measuring instrument further comprises a reporting unit that reports, in a case where the urine volume in the bladder compared by the comparator is greater than or equal to the second threshold, that the urine volume is greater than or equal to the second threshold.

**6.** The ultrasonic urine volume measuring instrument according to any one of Claims 1 to 3, further comprising:

a micturition input unit used by the measurement subject to input a micturition signal at a time the measurement subject feels micturition;
a measurement data memory that stores the urine volume in the bladder calculated by the arithmetic unit, its measurement time, and a micturition time corresponding to an input time of the micturition signal;
a maximum urine volume memory that stores maximum urine volume in the bladder that is a maximum value among urine volumes in the bladder calculated by the arithmetic unit in a certain period; and
a display unit that displays, at the time of inputting the micturition signal, retained urine volume upon micturition that is retained urine volume corresponding to the micturition time, and the maximum urine volume in the bladder.

**7.** The ultrasonic urine volume measuring instrument according to Claim 6, wherein the retained urine volume upon micturition is urine volume in the bladder calculated by the arithmetic unit in a certain time prior to the time of inputting the micturition signal.

**8.** The ultrasonic urine volume measuring instrument according to Claim 6, wherein the retained urine volume upon micturition is urine volume in the bladder newly calculated by the arithmetic unit after the ultrasound device transmits ultrasound at the time of inputting the micturition signal.

**9.** The ultrasonic urine volume measuring instrument according to Claim 6, further comprising:

a urination input unit used by the measurement subject to input a urination signal when the measurement subject urinates,
wherein the measurement data memory further stores a urination time corresponding to an input time of the urination signal input using the urination input unit.

**10.** The ultrasonic urine volume measuring instrument according to any one of Claims 6 to 9, wherein the display unit displays urine volume in the bladder used in at least one of toilet training and bladder training.

**11.** A urine volume management data generating and displaying method using the ultrasonic urine volume measuring instrument according to any one of Claims 6 to 10, comprising:

a step of generating, on the basis of urine volume in the bladder and its measurement time, and the micturition time, stored in the ultrasonic urine volume measuring instrument, urine volume management data by chronologically arranging at least urine-volume-in-bladder data including the urine volume in the bladder and its measurement time, and micturition data including the micturition time; and
a step of displaying at least part of the urine volume management data in the form of a table or a graph.

**12.** The method according to Claim 11, wherein the urine volume management data further includes urination time data corresponding to an input time of a urination signal input by the measurement subject when the measurement subject urinates.

**13.** The method according to Claim 12, further comprising:

a step of calculating the number of micturitions that is the number of micturition signals in a certain period on the basis of the micturition data, and the number of urinations that is the number of urination start signals or urination end signals in a certain period on the basis of the urination time data; and
a step of displaying the maximum urine volume in the bladder, the number of micturitions, and the number of urinations.

**14.** The method according to Claim 11, further comprising:

a step of searching the urine-volume-in-bladder data for a urine volume decrease time at which urine volume in the bladder decreases with time, and storing the amount of decrease as urination volume,
wherein the urine volume management data includes the urination volume.

**15.** The method according to Claim 14, further comprising:

a step of calculating a time difference between each urine volume decrease time and the urination time or the micturition time,
wherein the urine volume management data includes the time difference or data calculated from the time difference.

FIG.1

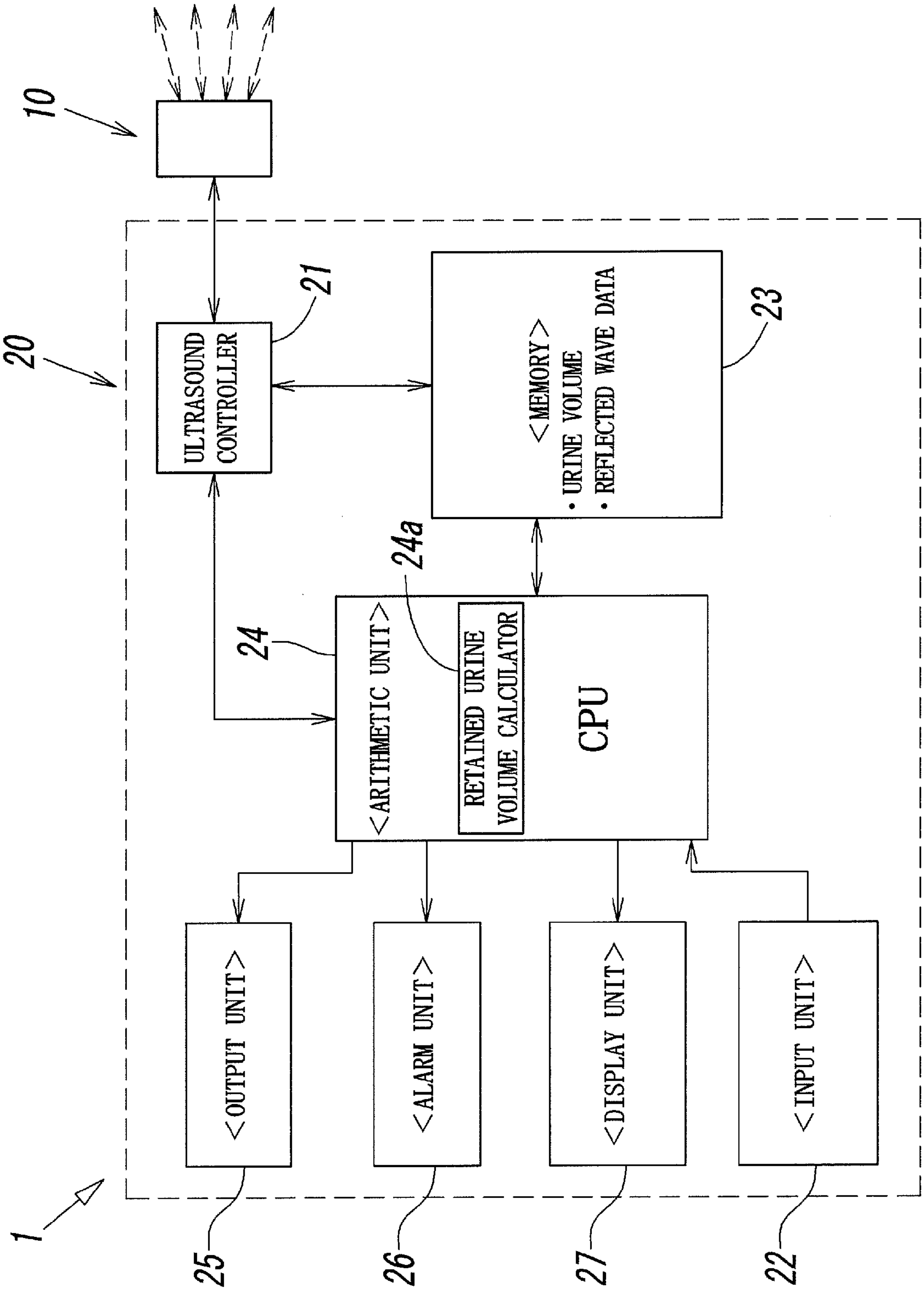
10
20
1
ULTRASOUND CONTROLLER
21
<MEMORY>
· URINE VOLUME
· REFLECTED WAVE DATA
23
24a
24
<ARITHMETIC UNIT>
RETAINED URINE VOLUME CALCULATOR
CPU
<OUTPUT UNIT>
<ALARM UNIT>
<DISPLAY UNIT>
<INPUT UNIT>
25
26
27
22

FIG.2

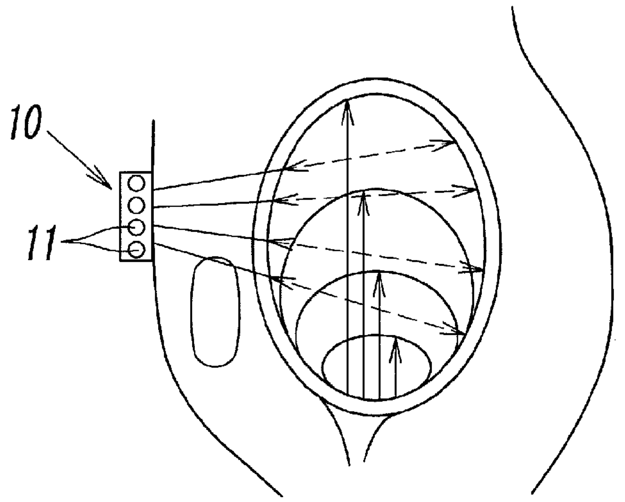


FIG.3

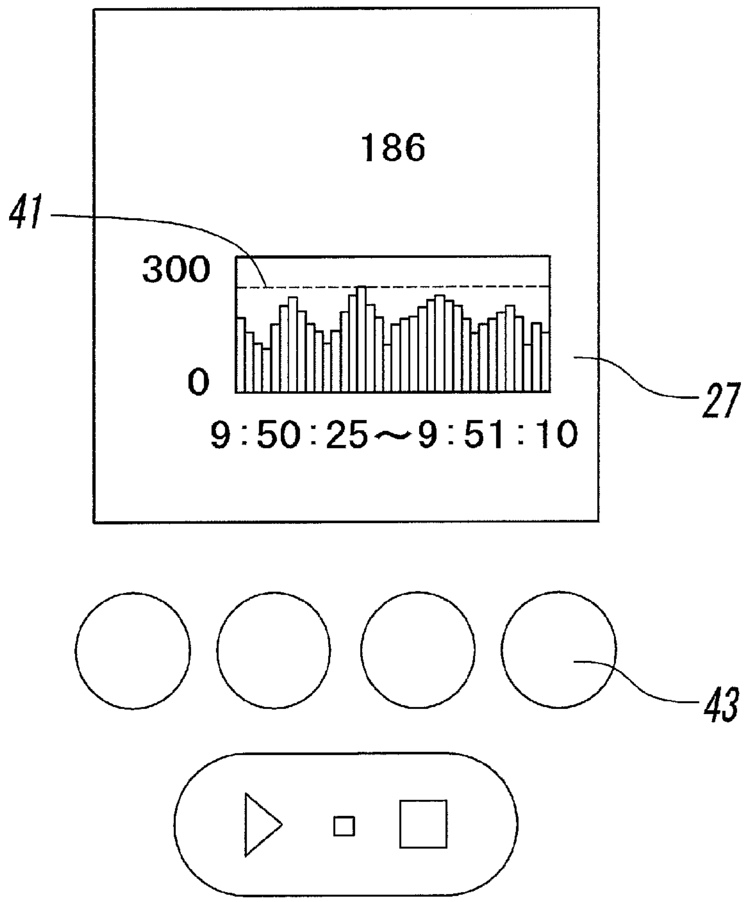

FIG.4

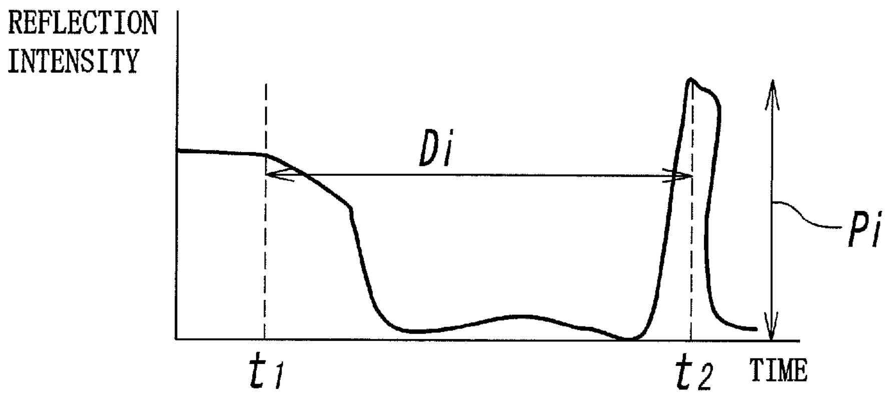
REFLECTION INTENSITY
Di
Pi
t1
t2
TIME

FIG.5

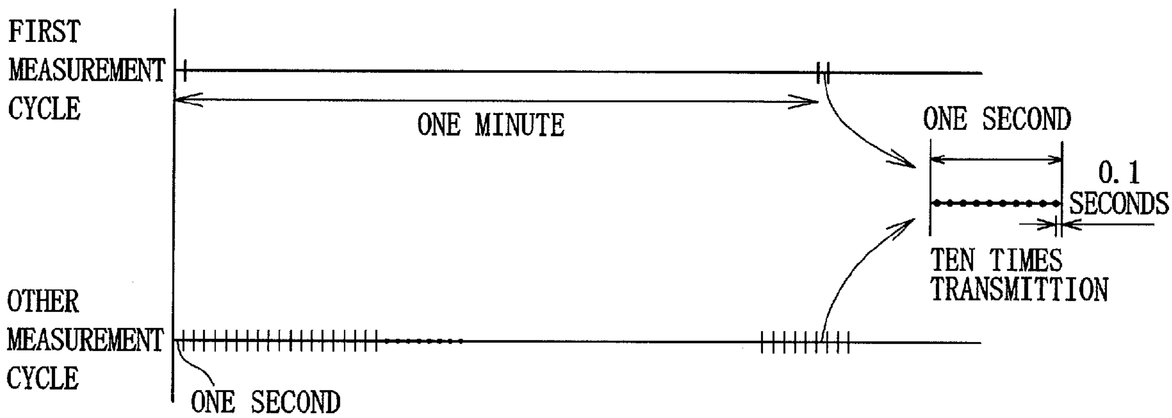
FIRST MEASUREMENT CYCLE
ONE MINUTE
ONE SECOND
0. 1 SECONDS
TEN TIMES TRANSMITTION
OTHER MEASUREMENT CYCLE
ONE SECOND

FIG.6

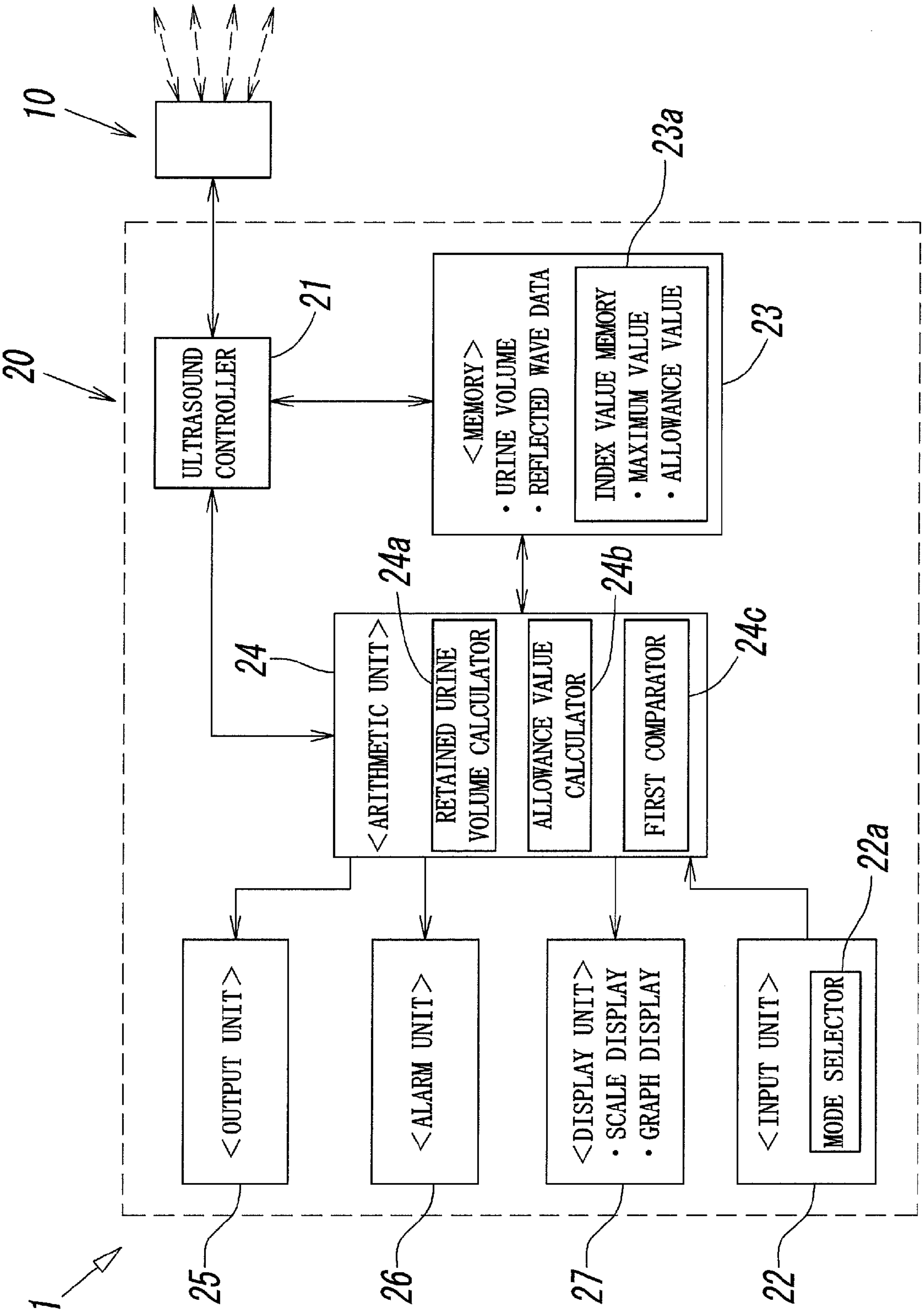
1
10
20
ULTRASOUND CONTROLLER
21
<MEMORY>
· URINE VOLUME
· REFLECTED WAVE DATA
INDEX VALUE MEMORY
· MAXIMUM VALUE
· ALLOWANCE VALUE
23a
23
24
<ARITHMETIC UNIT>
24a
RETAINED URINE VOLUME CALCULATOR
24b
ALLOWANCE VALUE CALCULATOR
24c
FIRST COMPARATOR
<OUTPUT UNIT>
25
<ALARM UNIT>
26
<DISPLAY UNIT>
· SCALE DISPLAY
· GRAPH DISPLAY
27
<INPUT UNIT>
MODE SELECTOR
22a
22

FIG.7

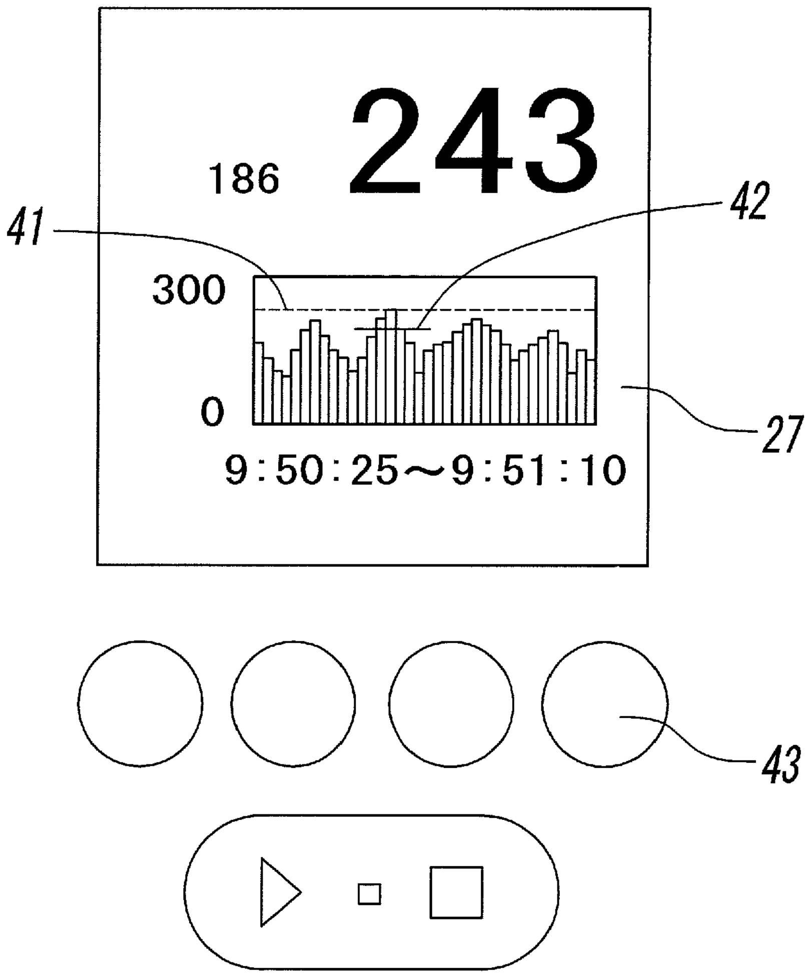

*FIG.8*

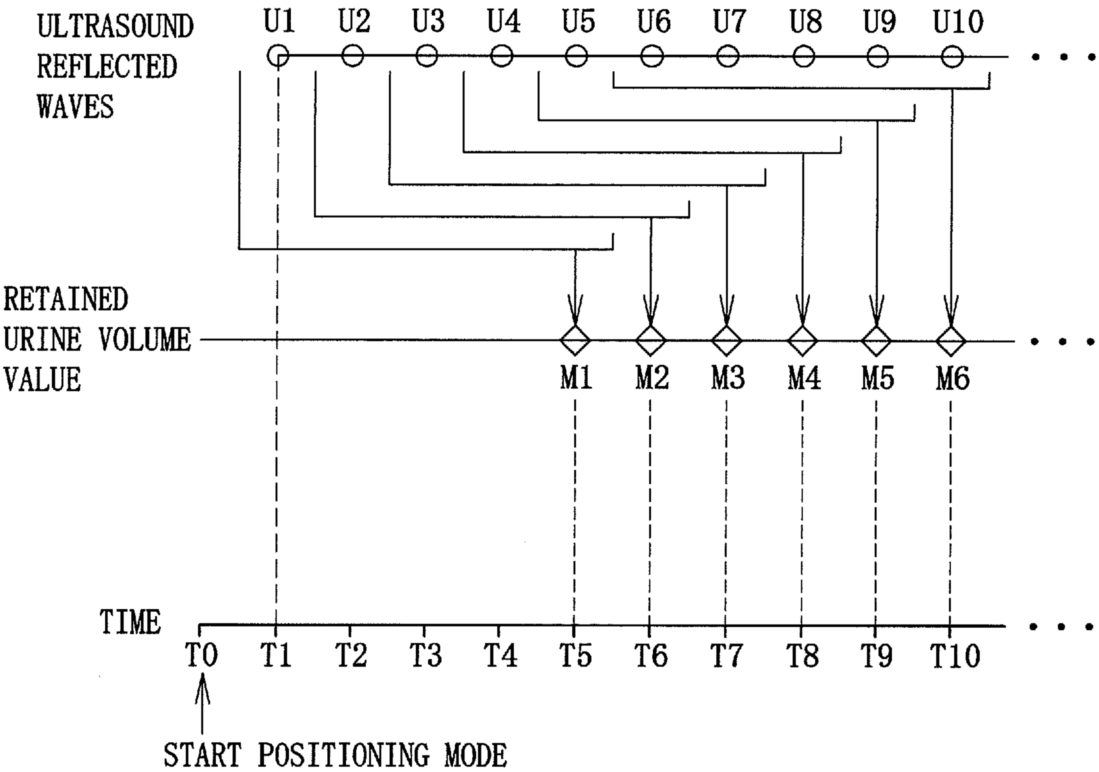
ULTRASOUND REFLECTED WAVES
U1 U2 U3 U4 U5 U6 U7 U8 U9 U10
RETAINED URINE VOLUME VALUE
M1 M2 M3 M4 M5 M6
TIME
T0 T1 T2 T3 T4 T5 T6 T7 T8 T9 T10
START POSITIONING MODE

FIG.9

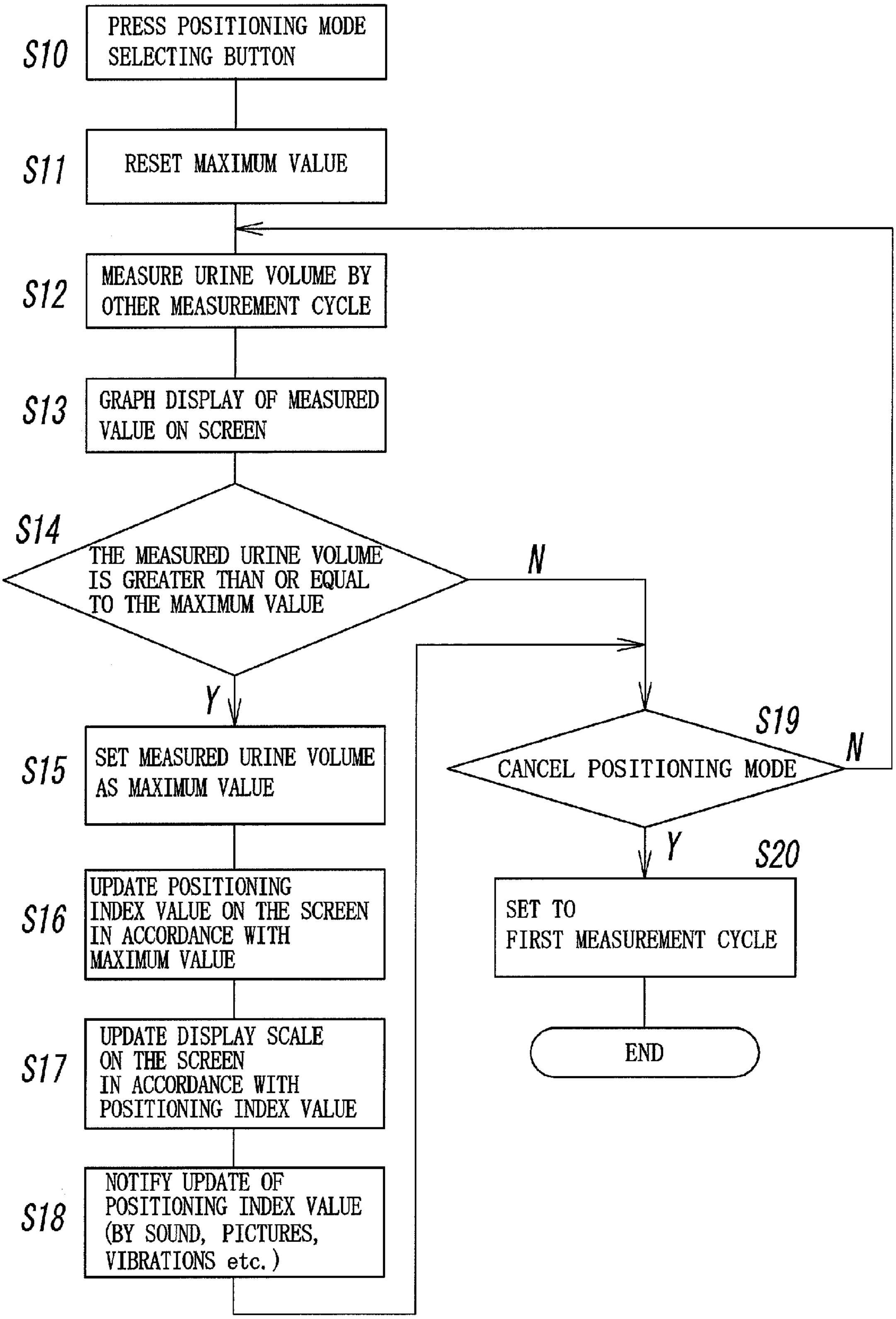
S10
PRESS POSITIONING MODE SELECTING BUTTON
S11
RESET MAXIMUM VALUE
S12
MEASURE URINE VOLUME BY OTHER MEASUREMENT CYCLE
S13
GRAPH DISPLAY OF MEASURED VALUE ON SCREEN
S14
THE MEASURED URINE VOLUME IS GREATER THAN OR EQUAL TO THE MAXIMUM VALUE
N
Y
S15
SET MEASURED URINE VOLUME AS MAXIMUM VALUE
S16
UPDATE POSITIONING INDEX VALUE ON THE SCREEN IN ACCORDANCE WITH MAXIMUM VALUE
S17
UPDATE DISPLAY SCALE ON THE SCREEN IN ACCORDANCE WITH POSITIONING INDEX VALUE
S18
NOTIFY UPDATE OF POSITIONING INDEX VALUE (BY SOUND, PICTURES, VIBRATIONS etc.)
S19
CANCEL POSITIONING MODE
N
Y
S20
SET TO FIRST MEASUREMENT CYCLE
END

FIG.10

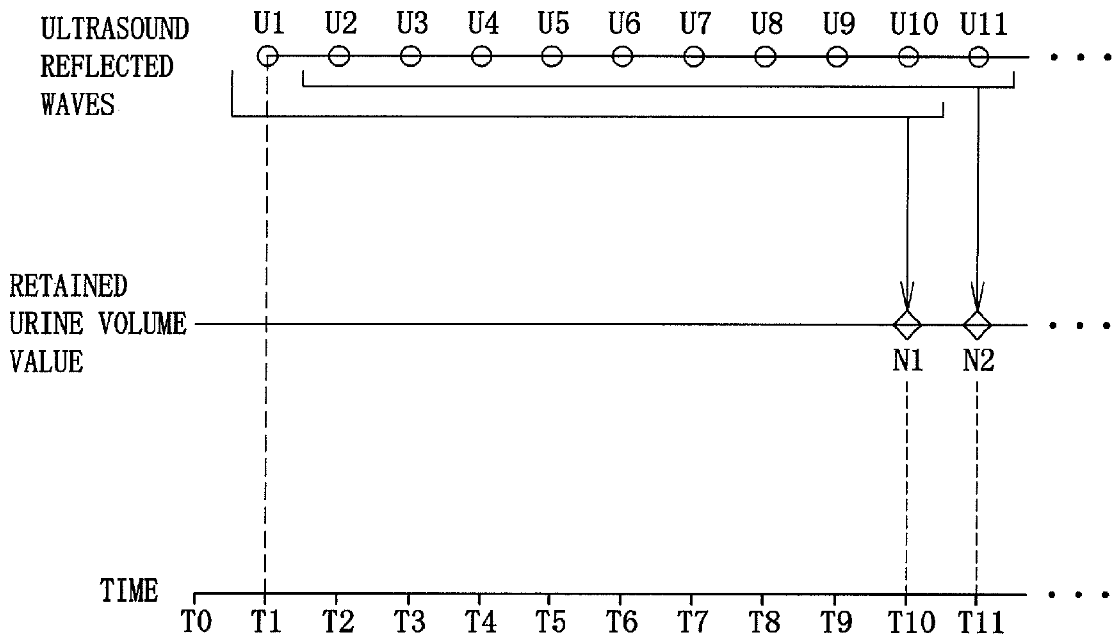
ULTRASOUND
REFLECTED
WAVES
U1 U2 U3 U4 U5 U6 U7 U8 U9 U10 U11
RETAINED
URINE VOLUME
VALUE
N1 N2
TIME
T0 T1 T2 T3 T4 T5 T6 T7 T8 T9 T10 T11

FIG.11

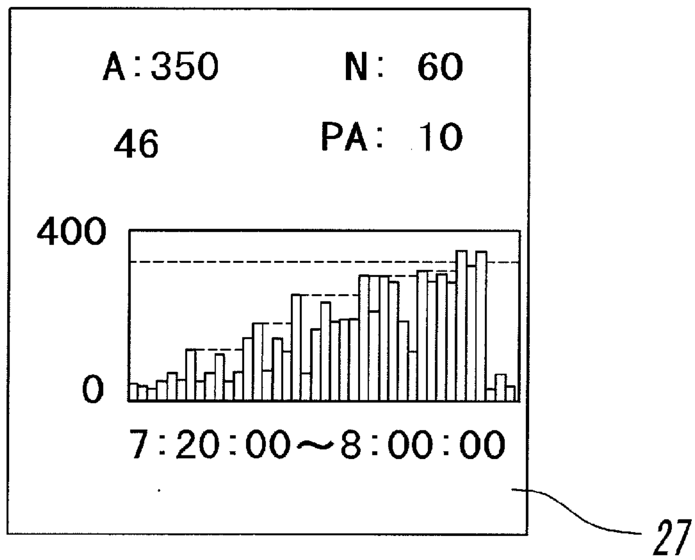
A:350
N: 60
46
PA: 10
400
0
7:20:00～8:00:00
27

FIG. 12

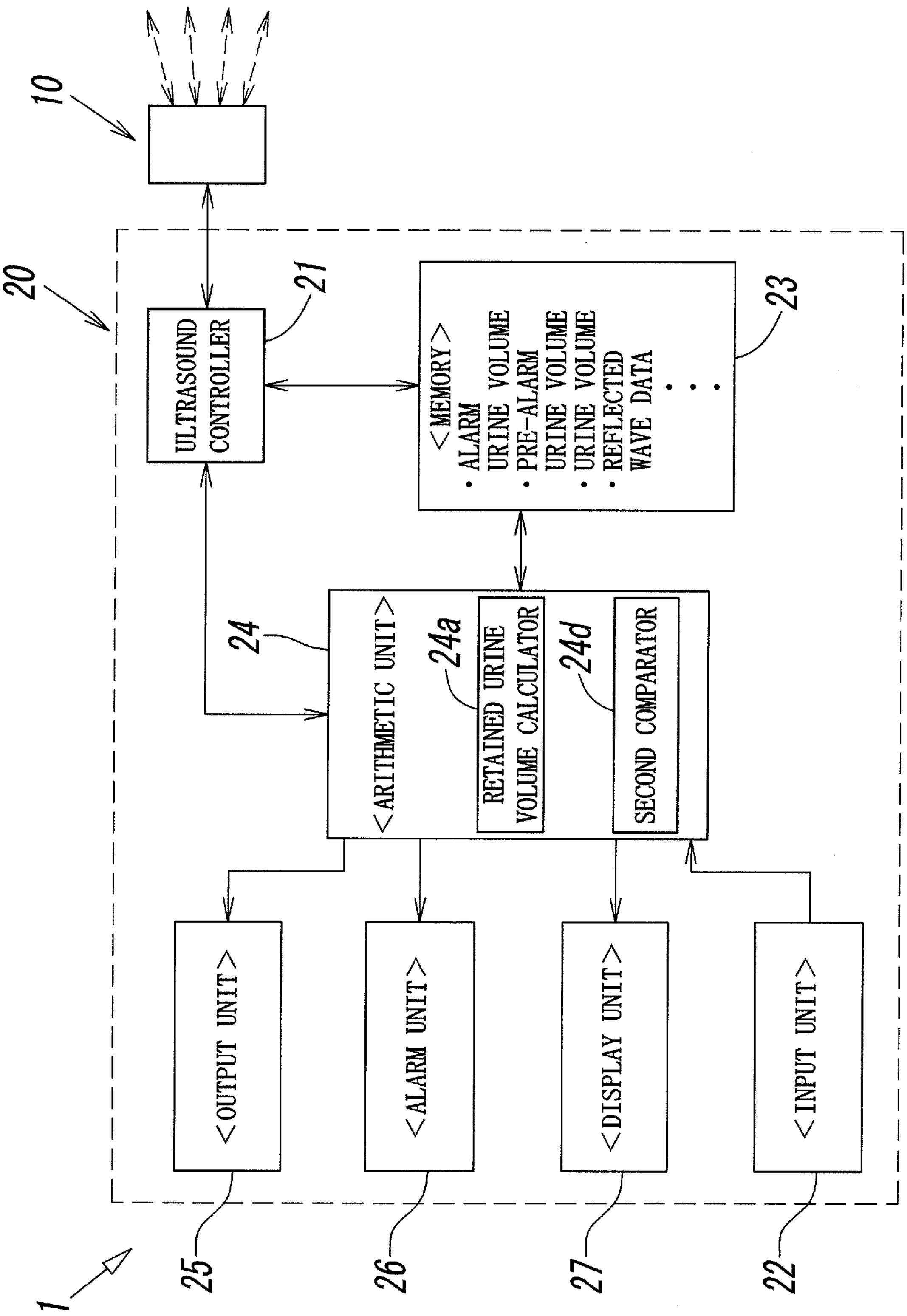
10
20
1
ULTRASOUND CONTROLLER
21
<MEMORY>
· ALARM URINE VOLUME
· PRE-ALARM URINE VOLUME
· URINE VOLUME
· REFLECTED WAVE DATA
...
23
24
<ARITHMETIC UNIT>
24a
RETAINED URINE VOLUME CALCULATOR
24d
SECOND COMPARATOR
<OUTPUT UNIT>
<ALARM UNIT>
<DISPLAY UNIT>
<INPUT UNIT>
25
26
27
22

FIG.13

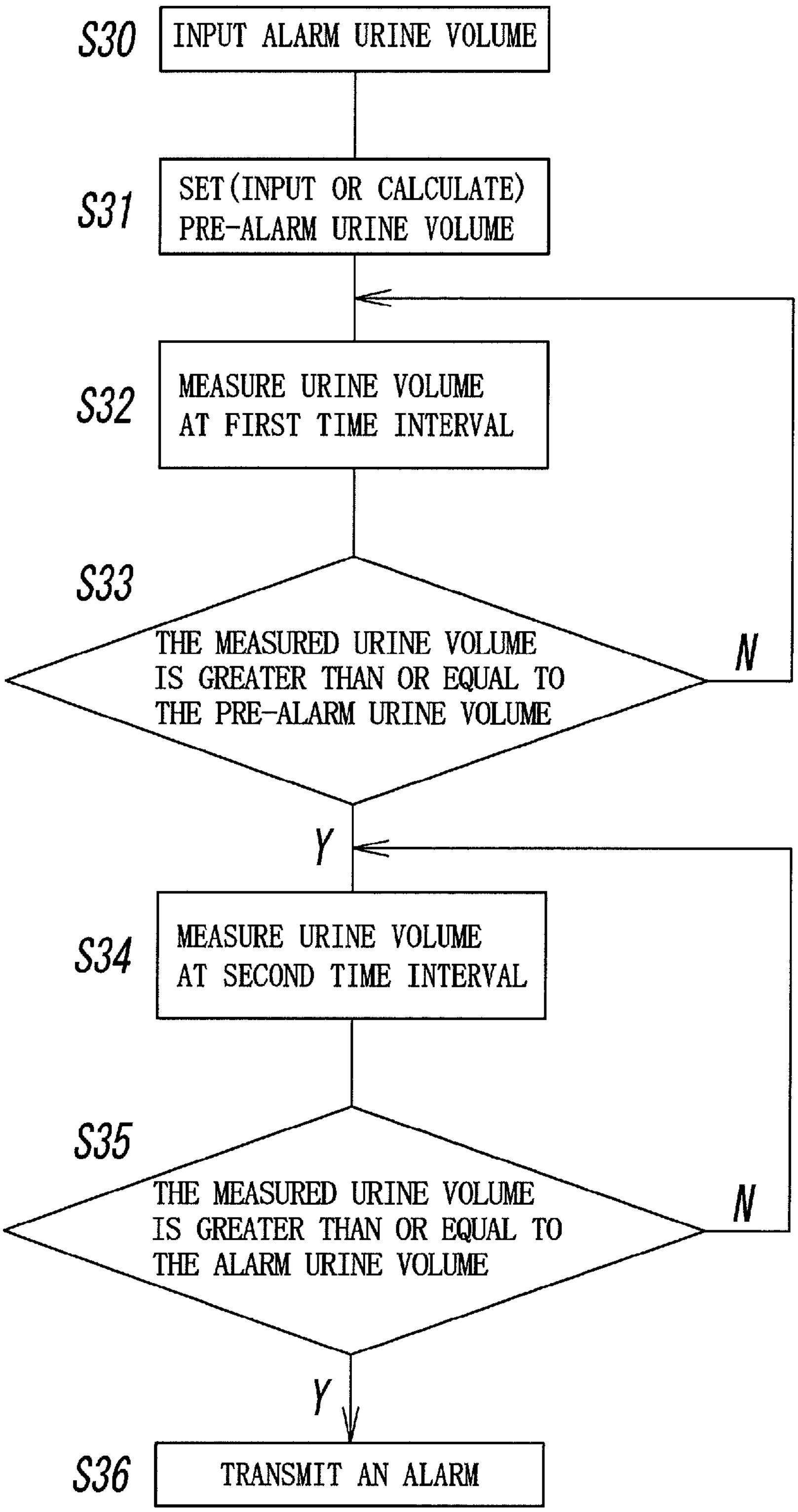
S30
INPUT ALARM URINE VOLUME
S31
SET(INPUT OR CALCULATE) PRE-ALARM URINE VOLUME
S32
MEASURE URINE VOLUME AT FIRST TIME INTERVAL
S33
THE MEASURED URINE VOLUME IS GREATER THAN OR EQUAL TO THE PRE-ALARM URINE VOLUME
N
Y
S34
MEASURE URINE VOLUME AT SECOND TIME INTERVAL
S35
THE MEASURED URINE VOLUME IS GREATER THAN OR EQUAL TO THE ALARM URINE VOLUME
N
Y
S36
TRANSMIT AN ALARM

FIG.14

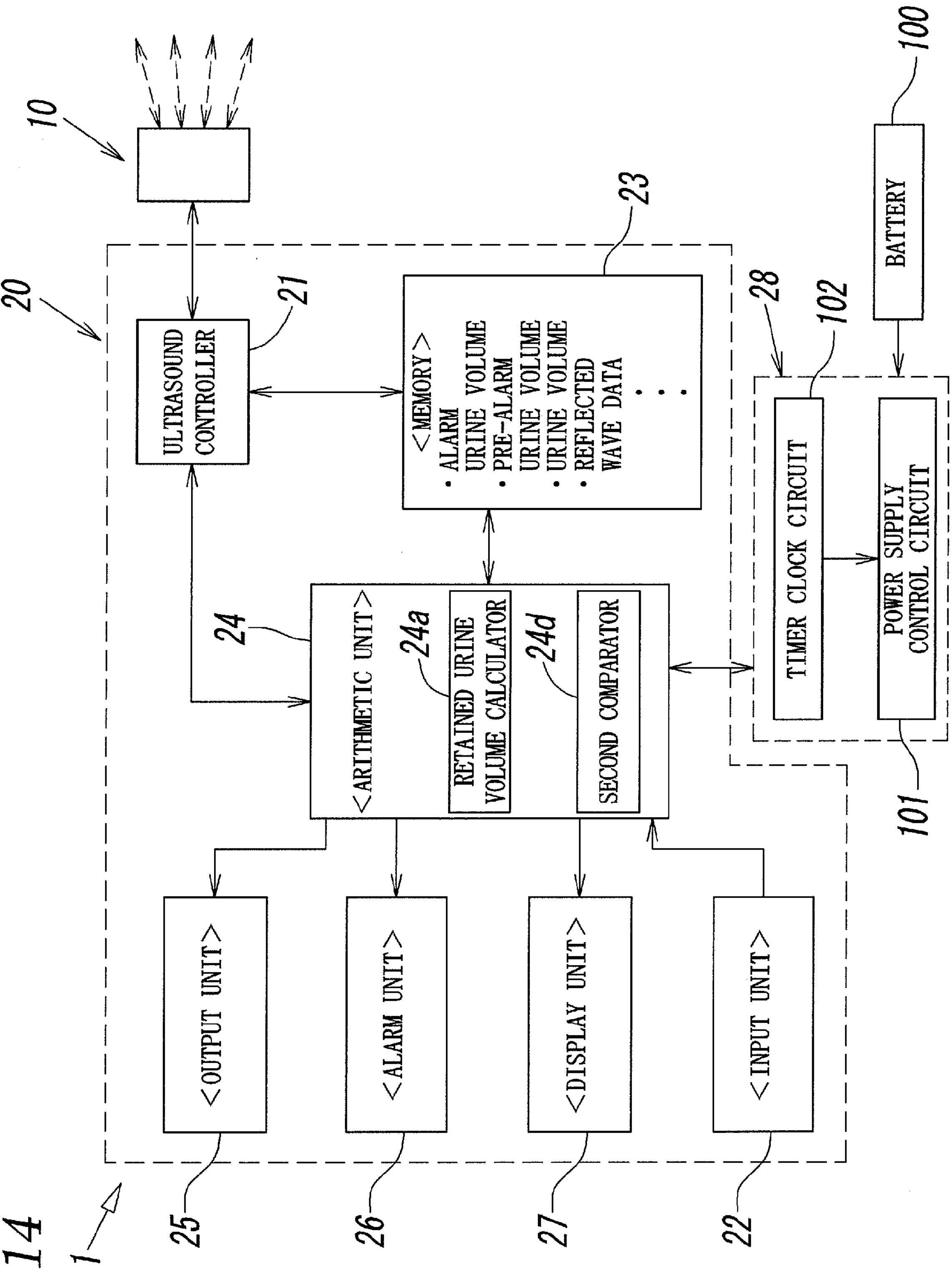
10
20
21
ULTRASOUND CONTROLLER
23
<MEMORY>
· ALARM URINE VOLUME
· PRE-ALARM URINE VOLUME
· URINE VOLUME
· REFLECTED WAVE DATA
...
BATTERY
100
28
102
TIMER CLOCK CIRCUIT
POWER SUPPLY CONTROL CIRCUIT
101
24
<ARITHMETIC UNIT>
24a
RETAINED URINE VOLUME CALCULATOR
24d
SECOND COMPARATOR
<OUTPUT UNIT>
<ALARM UNIT>
<DISPLAY UNIT>
<INPUT UNIT>
1
25
26
27
22

FIG.15

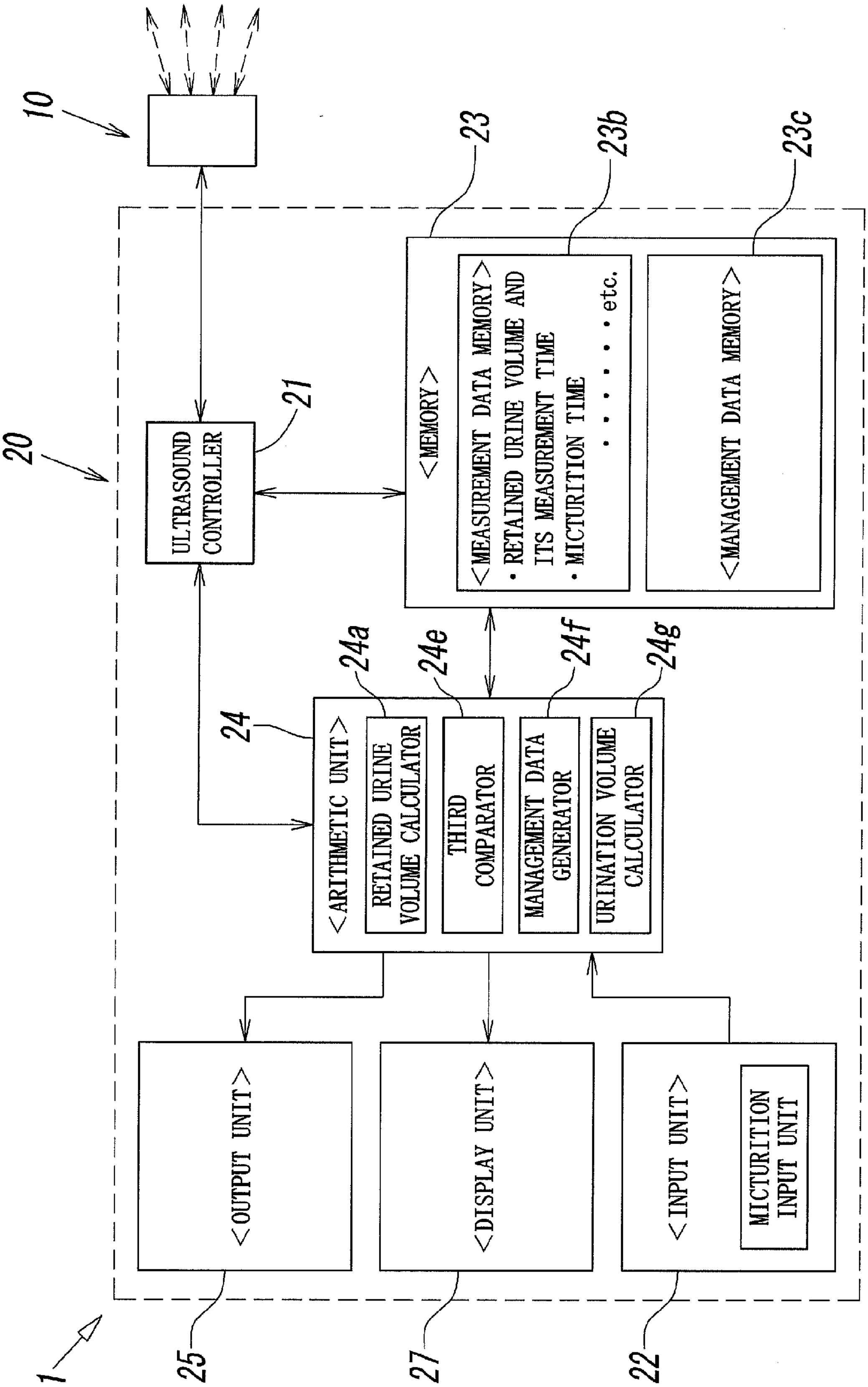
10
20
21
ULTRASOUND CONTROLLER
23
23b
23c
<MEMORY>
<MEASUREMENT DATA MEMORY>
· RETAINED URINE VOLUME AND ITS MEASUREMENT TIME
· MICTURITION TIME
· · · · · · etc.
<MANAGEMENT DATA MEMORY>
24
24a
24e
24f
24g
<ARITHMETIC UNIT>
RETAINED URINE VOLUME CALCULATOR
THIRD COMPARATOR
MANAGEMENT DATA GENERATOR
URINATION VOLUME CALCULATOR
<OUTPUT UNIT>
<DISPLAY UNIT>
<INPUT UNIT>
MICTURITION INPUT UNIT
1
25
27
22

FIG.16

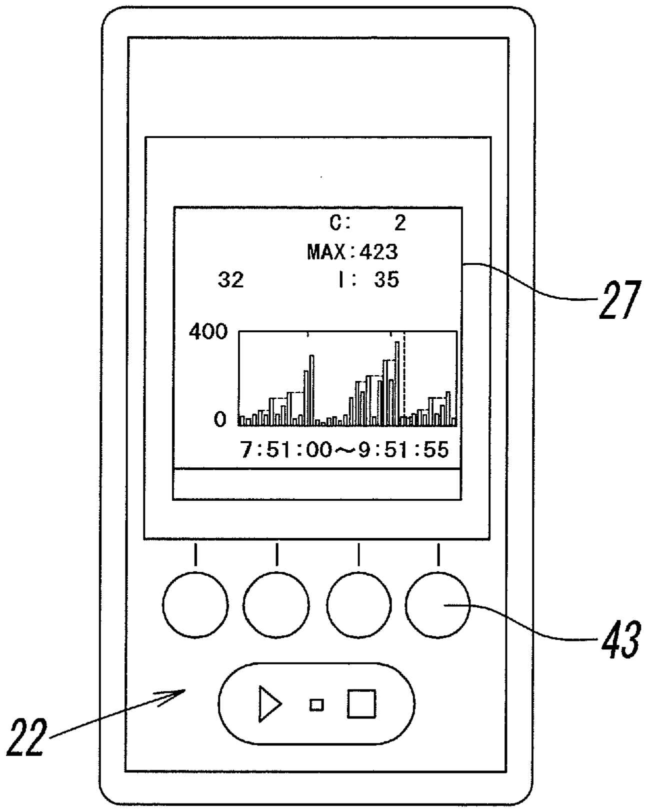

*FIG.17*

| TIME | URINE VOLUME IN THE BLADDER |
|---|---|
| 9:30:00 | 45 |
| 9:31:00 | 47 |
| 9:32:00 | 45 |
| 9:33:00 | 48 |
| 9:34:00 | 48 |
| 9:35:00 | 51 |
| 9:36:00 | 53 |
| 9:37:00 | 57 |
| . . . . | . . . . |
| 11:09:00 | 403 |
| 11:10:00 | 405 |
| 11:11:00 | 406 |
| 11:12:00 | 405 |
| 11:13:00 | 408 |
| 11:14:00 | 407 |
| 11:15:00 | 407 |
| 11:16:00 | 409 |
| 11:17:00 | 411 |
| 11:18:00 | 413 |
| 11:19:00 | 417 |
| 11:20:00 | 419 |
| 11:21:00 | 420 |
| 11:22:00 | 420 |
| 11:23:00 | 421 |
| 11:24:00 | 43 |
| 11:25:00 | 45 |
| 11:26:00 | 49 |

FIG.18

| MICTURITION TIME |
|---|
| 9:41:16 |
| 11:17:38 |

FIG.19A

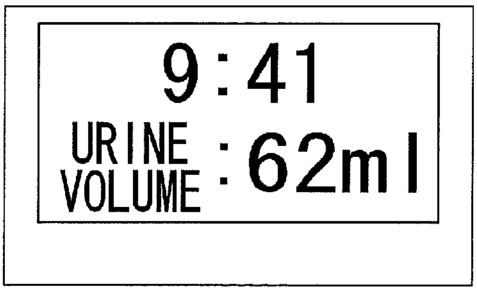


FIG.19B

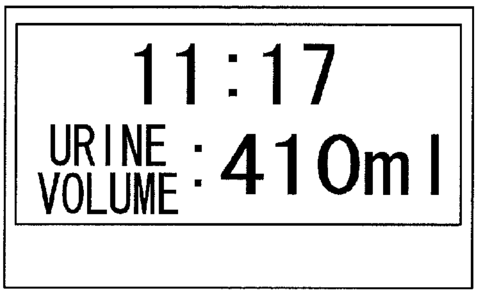

*FIG.20*

| TIME | URINATION VOLUME | URINE VOLUME IN THE BLADDER | MICTURITION |
|---|---|---|---|
| 9:40:00 | | 60 | |
| 9:41:00 | | 66 | |
| 9:41:16 | | | ○ |
| 9:42:00 | | 71 | |
| 9:43:00 | | 75 | |
| 9:44:00 | | 78 | |
| 9:45:00 | | 81 | |
| 9:46:00 | | 85 | |
| 9:47:00 | | 88 | |
| 9:48:00 | | 92 | |
| 9:49:00 | | 97 | |
| ⋮ | ⋮ | ⋮ | |
| 11:14:00 | | 407 | |
| 11:15:00 | | 407 | |
| 11:16:00 | | 409 | |
| 11:17:00 | | 411 | |
| 11:17:38 | | | ○ |
| 11:18:00 | | 413 | |
| 11:19:00 | | 417 | |
| 11:20:00 | | 419 | |
| 11:21:00 | | 420 | |
| 11:22:00 | | 420 | |
| 11:23:00 | | 421 | |
| 11:24:00 | 378 | 43 | |
| 11:25:00 | | 45 | |
| 11:26:00 | | 49 | |

MAX VALUE 423

*FIG.21*

| TIME | URINATION VOLUME | URINE VOLUME IN THE BLADDER | MICTURITION |
|---|---|---|---|
| 9:37:00 | | 57 | |
| 9:38:00 | | 59 | |
| 9:39:00 | | 58 | |
| 9:40:00 | | 60 | |
| 9:41:00 | | 66 | ○ |
| 9:42:00 | | 71 | |
| 9:43:00 | | 75 | |
| 9:44:00 | | 78 | |
| ⋮ | ⋮ | ⋮ | |
| 11:10:00 | | 405 | |
| 11:11:00 | | 406 | |
| 11:12:00 | | 405 | |
| 11:13:00 | | 408 | |
| 11:14:00 | | 407 | |
| 11:15:00 | | 407 | |
| 11:16:00 | | 409 | |
| 11:17:00 | | 411 | ○ |
| 11:18:00 | | 413 | |
| 11:19:00 | | 417 | |
| 11:20:00 | | 419 | |
| 11:21:00 | | 420 | |
| 11:22:00 | | 420 | |
| 11:23:00 | | 421 | |
| 11:24:00 | 378 | 43 | |
| 11:25:00 | | 45 | |
| 11:26:00 | | 49 | |

MAX VALUE 423

FIG.22

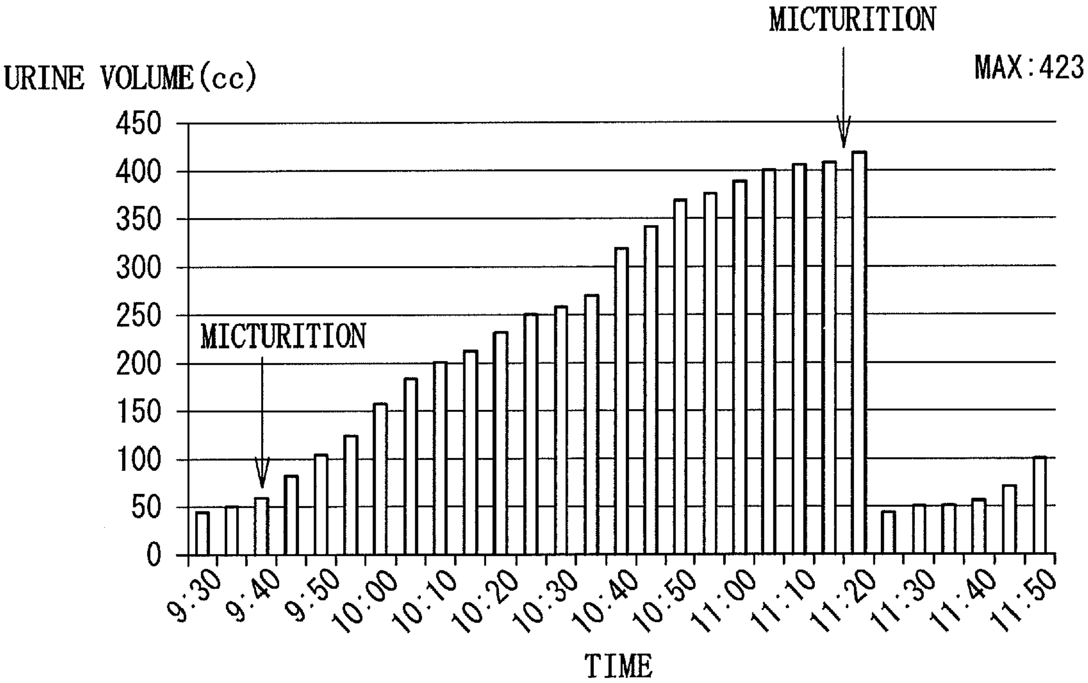
MICTURITION
MAX:423
URINE VOLUME(cc)
450
400
350
300
250
200
150
100
50
0
MICTURITION
9:30
9:40
9:50
10:00
10:10
10:20
10:30
10:40
10:50
11:00
11:10
11:20
11:30
11:40
11:50
TIME

*FIG.23*

| TIME | URINATION VOLUME | URINE VOLUME IN THE BLADDER | MICTURITION |
|---|---|---|---|
| 9:41 | | 64 | ○ |
| 11:17 | | 410 | ○ |
| 11:23 | 378 | 43 | |

MAX VALUE 423

*FIG.24*

| TIME | URINATION VOLUME | URINE VOLUME IN THE BLADDER | MICTURITION |
|---|---|---|---|
| 9:41 | | 64 | ○ |
| 11:17 | | 410 | ○ |
| 11:22 | | 421 | |
| 11:23 | 378 | 43 | |

MAX VALUE 423

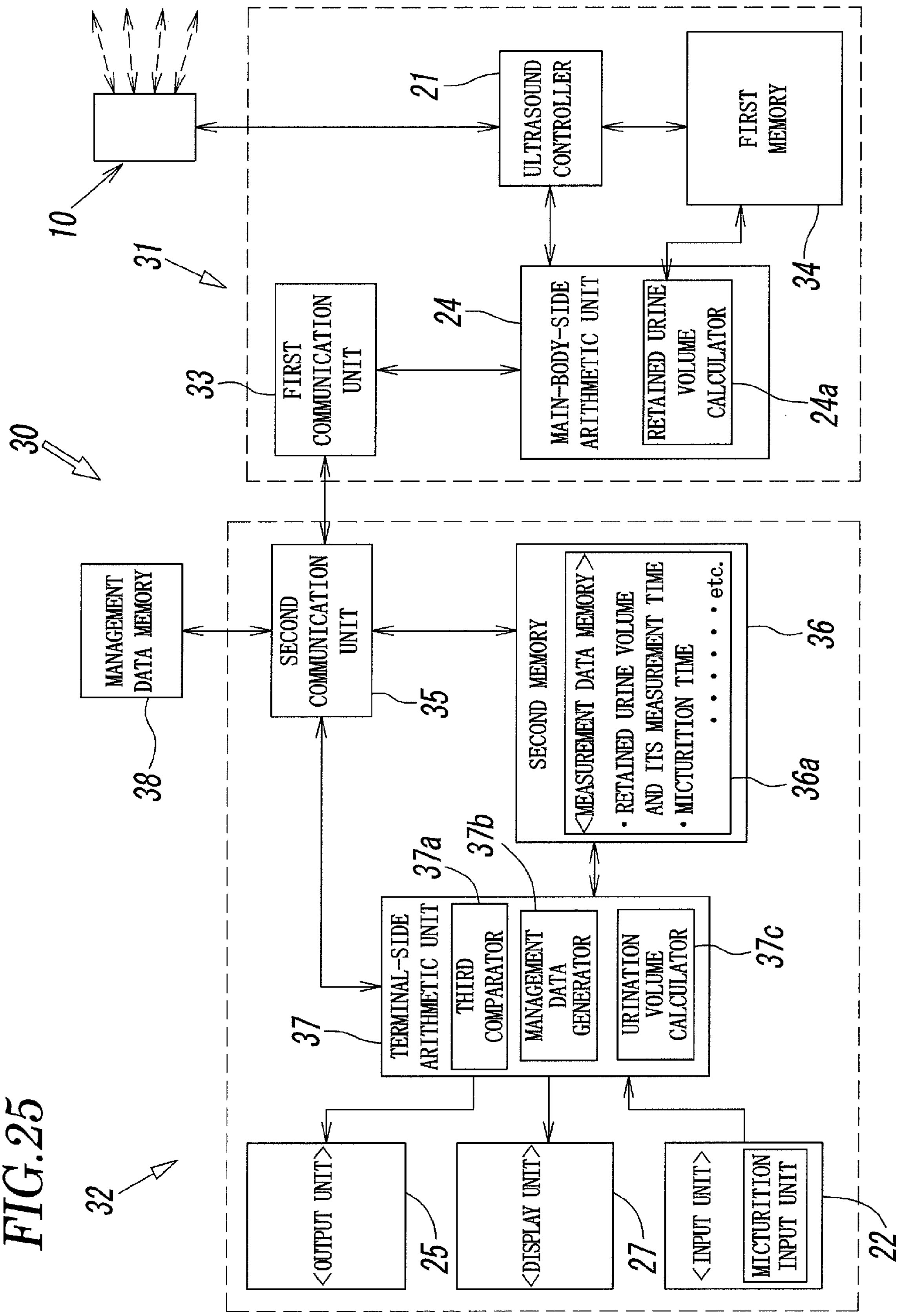
10
30
31
21
ULTRASOUND CONTROLLER
FIRST MEMORY
34
33
FIRST COMMUNICATION UNIT
24
MAIN-BODY-SIDE ARITHMETIC UNIT
RETAINED URINE VOLUME CALCULATOR
24a
MANAGEMENT DATA MEMORY
38
SECOND COMMUNICATION UNIT
35
SECOND MEMORY
<MEASUREMENT DATA MEMORY>
· RETAINED URINE VOLUME AND ITS MEASUREMENT TIME
· MICTURITION TIME
· · · · · · · etc.
36
36a
37a
37b
TERMINAL-SIDE ARITHMETIC UNIT
THIRD COMPARATOR
MANAGEMENT DATA GENERATOR
URINATION VOLUME CALCULATOR
37
37c
32
<OUTPUT UNIT>
25
<DISPLAY UNIT>
27
<INPUT UNIT>
MICTURITION INPUT UNIT
22

FIG.25

*FIG.26*

| | | |
|---|---|---|
| MAXIMUM URINE VOLUME IN THE BLADDER | FROM APRIL 1 TO APRIL 3 | 382ml |
| NUMBER OF URINATIONS | APRIL 3 | 10 TIMES |
| URINATION VOLUME | APRIL 3 | 1,400ml |
| NUMBER OF MICTURITIONS | APRIL 3 | 14 TIMES |

Europäisches Patentamt
European Patent Office
Office européen des brevets

# EUROPEAN SEARCH REPORT

Application Number
EP 15 18 2524

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2007/123778 A1 (KANTOROVICH EDWARD [IL]) 31 May 2007 (2007-05-31)<br>* paragraphs [0118] - [0120] *<br>* paragraphs [0167] - [0177] *<br>* paragraphs [0196] - [0235] *<br>* claims; figures * | 1,5-15<br>2-4 | INV.<br>A61B8/00<br>A61B8/08 |
| Y | US 5 058 591 A (COMPANION JOHN A [US] ET AL) 22 October 1991 (1991-10-22)<br>* column 6, line 13 - column 7, line 38 *<br>* column 9, lines 13-37 * | 2-4 | |
| A | EP 2 186 483 A1 (DIAGNOSTIC ULTRASOUND EUROP B [NL]) 19 May 2010 (2010-05-19)<br>* paragraphs [0032] - [0041] *<br>* figures * | 1-15 | |
| A | US 6 359 190 B1 (TER-OVANESYAN EVGENY [US] ET AL) 19 March 2002 (2002-03-19)<br>* column 9, line 64 - column 10, line 13 * | 1-15 | |
| | | | TECHNICAL FIELDS SEARCHED (IPC) |
| A,D | JP 2011 183142 A (UNIV NAGASAKI; SCIENCE RES CO LTD; ONGA ENG KK) 22 September 2011 (2011-09-22)<br>* the whole document * | 1-15 | A61B |
| A | JP 2007 233949 A (UNIV KYOTO) 13 September 2007 (2007-09-13)<br>* the whole document * | 1-15 | |

1

The present search report has been drawn up for all claims

| Place of search | Date of completion of the search | Examiner |
|---|---|---|
| The Hague | 27 October 2015 | Bataille, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 18 2524

This annex lists the patent family members relating to the patent documents cited in the above-mentioned European search report. The members are as contained in the European Patent Office EDP file on
The European Patent Office is in no way liable for these particulars which are merely given for the purpose of information.

27-10-2015

| Patent document cited in search report | Publication date | Patent family member(s) | Publication date |
|---|---|---|---|
| US 2007123778 A1 | 31-05-2007 | EP 1682005 A2 | 26-07-2006 |
| | | JP 2007508857 A | 12-04-2007 |
| | | US 2007123778 A1 | 31-05-2007 |
| | | WO 2005034717 A2 | 21-04-2005 |
| US 5058591 A | 22-10-1991 | NONE | |
| EP 2186483 A1 | 19-05-2010 | AU 2003254372 A1 | 11-03-2004 |
| | | EP 1551305 A1 | 13-07-2005 |
| | | EP 2186483 A1 | 19-05-2010 |
| | | GB 2391625 A | 11-02-2004 |
| | | JP 4549853 B2 | 22-09-2010 |
| | | JP 5443883 B2 | 19-03-2014 |
| | | JP 2005535420 A | 24-11-2005 |
| | | JP 2009279435 A | 03-12-2009 |
| | | US 2005215896 A1 | 29-09-2005 |
| | | US 2006111633 A1 | 25-05-2006 |
| | | US 2008139934 A1 | 12-06-2008 |
| | | US 2010198075 A1 | 05-08-2010 |
| | | WO 2004017834 A1 | 04-03-2004 |
| US 6359190 B1 | 19-03-2002 | NONE | |
| JP 2011183142 A | 22-09-2011 | NONE | |
| JP 2007233949 A | 13-09-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 4677615 B
- JP 2009512532 A
- JP 2000210286 A
- US 6565512 B
- JP 2007508857 A
- JP 2011183142 A
- JP 2014023813 A